# EUROPÄISCHE PATENTANMELDUNG

(11) **EP 4 666 979 A1**
(43) Veröffentlichungstag der Anmeldung: **24.12.2025**
(21) Anmeldenummer: 24182861.5
(22) Anmeldetag: 18.06.2024
(51) Int. Cl.: A61B 34/30

(54) **TECHNIK ZUR ANSTEUERUNG EINER MOTORISCHEN UNTERSTÜTZUNG EINER BEWEGUNG EINER MEDIZINTECHNISCHEN KOMPONENTE**

(71) Anmelder: Siemens Healthineers AG, 91301 Forchheim (DE)
(72) Erfinder: Deinlein, Andreas, 95447 Bayreuth (DE); Friedrich, Stefan, 96135 Stegaurach (DE); Gambke, Günther, 95466 Weidenberg (DE); Schmidt, Verena, 92681 Erbendorf (DE)
(74) Vertreter: Siemens Healthineers Patent Attorneys

(57) **Zusammenfassung**

Die Erfindung betriff eine Technik zum Ansteuern einer Antriebseinheit (306) zur motorischen Unterstützung einer Bewegung einer mobilen medizintechnischen Komponente (708). Ein computer-implementiertes Verfahren umfasst ein Erfassen (S102) von Kraftmessdaten, die eine Kraft repräsentieren, die zur Bewegungssteuerung der medizintechnischen Komponente (708) an einem Bedienelement, insbesondere einem Handgriff (702), an der medizintechnischen Komponente (708) aufgebracht wird. Mittels eines neuronalen Netzwerks werden die erfassten (S102) Kraftmessdaten zum Bestimmen (S108) von Steuerdaten zur Ansteuerung der Antriebseinheit (306) zur motorischen Unterstützung der Bewegung der medizintechnischen Komponente (708) verarbeitet (S106). Die Antriebseinheit (306) wird mittels den bestimmten (S108) Steuerdaten angesteuert (S110).

## Beschreibung

Es wird eine Technik zum Ansteuern einer Antriebseinheit zur motorischen Unterstützung einer Bewegung einer medizintechnischen Komponente bereitgestellt. Die Technik umfasst insbesondere ein Verfahren, einen Controller, ein System, ein Computerprogrammprodukt und ein computerlesbares Speichermedium.

Bei einer kraftunterstützten Bewegungsansteuerung benutzt der Bediener einen oder mehrere Kraftsensoren, um ein oder mehrere motorisierte Achsen eines medizinischen Geräts anzusteuern und damit das Gerät zu bewegen. Das bedeutet, der Bediener gibt die Richtung und ein Ziel vor und die Bewegung wird "nur" motorisch unterstützt. Dies ist bei medizinischen Geräten oder deren medizintechnischen Komponenten regelmäßig notwendig, da aufgrund des Gewichts des Gerätes bzw. der Komponente eine komplett manuelle Bewegung für den Bediener nur schwer möglich wäre. Beispiele für medizintechnischen Komponenten bzw. medizinische Geräte, bei denen eine motorische Unterstützung sinnvoll oder sogar notwendig ist, sind eine Patientenliege, einen C-Bogen oder ein mobiles bildgebendes Gerät wie ein (z.B. Kopf-) Computertomograph (CT) oder ein mobiles Röntgengerät.

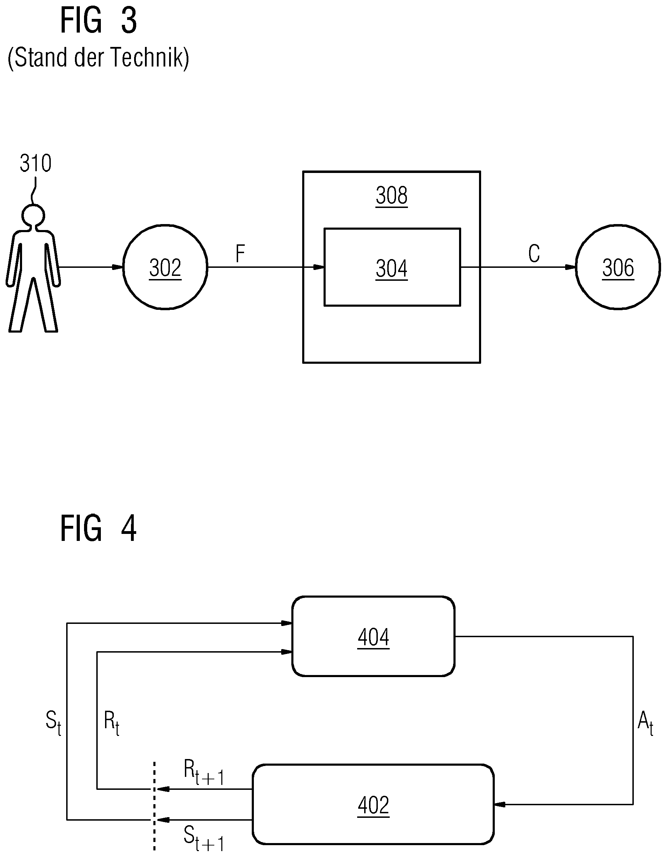

Fig. 3 zeigt schematisch vereinfacht einen Bediener 310, der eine Kraft F ausübt, die mittels eines Kraftsensors 302 gemessen wird. Der Kraftsensor 302 ist z.B. in einen Bediengriff integriert, und die Umsetzung der einwirkenden Kraft F auf eine Ansteuerung C eines oder mehrerer Motoren 306 erfolgt in mittels eines konventionellen Bewegungssteuerungsalgorithmus (auch: SW-Algorithmus) 304 auf einem Steuerungssystem 308.

Die Qualität der Ansteuerung hängt konventionellerweise stark von der Qualität der Krafteinwirkung des Bedieners ab. Wenn der Bediener erfahren ist, wird die Bewegungsführung vermutlich sehr effizient durchgeführt werden. Wenn hingegen ein unerfahrener Bediener die Krafteinwirkung vorgibt, kann es zu ruckartigen, ineffizienten Bewegungen kommen und der durchzuführende Workflow deutlich verlängert werden. Im ungünstigsten Fall ist der Bediener überhaupt nicht in der Lage das Gerät zu bedienen, etwa bei kleinen, "schwachen" Personen.

Bei der kraftunterstützten Bewegungsansteuerung ist die Effizienz des resultierenden medizinischen Workflows konventionellerweise stark von der Erfahrung und den Fähigkeiten des Bedieners abhängig, sowie von den Parametern des Bewegungssteuerungsalgorithmus bzw. des Steuerungssystems, welcher bzw. welches die gemessenen Kräfte in Bewegungen übersetzt. Das Problem der Parameterabhängigkeit sowie der Abhängigkeit vom Bediener konnte konventionellerweise nicht generisch gelöst werden. Die Sensorik sowie die Parametrisierung wird konventionellerweise auf einen Durchschnittsbediener ausgelegt.

Konventionellerweise werden Bewegungssteuerungsalgorithmen, wie lineare Verstärkung oder modell-basierte Admittanzregelung, eingesetzt, welche abhängig von den eingestellten Parametern, bei einer einwirkenden Kraft F immer die gleiche Ansteuerung C erzeugen. Denkbar wäre, dass der Bewegungssteuerungsalgorithmus, welcher die Krafteinwirkung auf die motorische Ansteuerung umsetzt, parametrisierbar ist und hierüber auf die Fähigkeit unterschiedlicher Bediener angepasst werden kann. Dies könnte prinzipiell manuell durch Konfiguration oder automatisch durch Erkennung des Bedieners erfolgen. Dennoch wäre es sehr komplex für normale Bediener, die richtige Anpassung des Bewegungssteuerungsalgorithmus zu finden. Zudem wechseln die Bediener im Krankenhaus häufig, wodurch dieser Ansatz in der Praxis nicht wirklich praktikabel wäre.

Es ist daher ein Ziel der vorliegenden Erfindung, eine Lösung für eine Optimierung einer motorischen Unterstützung der Bewegung einer medizintechnischen Komponente bereitzustellen. Die Optimierung kann sich beispielsweise auf eine Zeiteffizienz und/oder sanfte Bewegung beziehen. Alternativ oder ergänzend besteht die Aufgabe, eine Sicherheit eines Patienten beim Transport auf einer motorisch unterstützen Patientenliege zu verbessern und/oder einen Verschleiß der Antriebseinheit zur motorischen Unterstützung (z.B. des Motors, der Räder und/oder Bremsen) der medizintechnischen Komponente zu minimieren.

Diese Aufgabe wird gelöst durch ein Verfahren zum Ansteuern einer Antriebseinheit zur motorischen Unterstützung einer Bewegung einer medizintechnischen Komponente, durch einen Controller, durch ein System, durch ein Computerprogramm (und/der Computerprogrammprodukt) und durch ein Speichermedium gemäß den beigefügten unabhängigen Ansprüchen. Vorteilhafte Aspekte, Merkmale und Ausführungsformen sind in den abhängigen Ansprüchen und in der folgenden Beschreibung zusammen mit Vorteilen beschrieben.

Im Folgenden wird die erfindungsgemäße Lösung in Bezug auf das beanspruchte Verfahren zum Ansteuern einer Antriebseinheit zur motorischen Unterstützung einer Bewegung einer medizintechnischen Komponente sowie in Bezug auf den beanspruchten Controller beschrieben. Merkmale, Vorteile oder alternative Ausführungsformen hierin können den anderen beanspruchten Gegenständen (z.B. dem System, dem Computerprogramm oder einem Computerprogrammprodukt) zugeordnet werden und umgekehrt. Mit anderen Worten: Die Ansprüche für den Controller und/oder das System umfassend den Controller können durch Merkmale verbessert werden, die im Zusammenhang mit den Verfahren beschrieben oder beansprucht werden. In diesem Fall werden die funktionalen Merkmale des Verfahrens durch Struktureinheiten des Systems verkörpert und umgekehrt.

Gemäß einem Verfahrensaspekt wird ein (insbesondere computer-implementiertes) Verfahren zum Ansteuern einer Antriebseinheit zur motorischen Unterstützung einer Bewegung einer medizintechnischen Komponente bereitgestellt. Das Verfahren umfasst einen Schritt des Erfassens von Kraftmessdaten. Die Kraftmessdaten repräsentieren eine Kraft, die zur Bewegungssteuerung einer mobilen motorisch unterstützten medizintechnischen Komponente an einem Bedienelement (insbesondere einem Handgriff) an der medizintechnischen Komponente aufgebracht wird. Das Verfahren umfasst ferner einen Schritt des Verarbeitens, mittels eines neuronalen Netzwerks (NN), der erfassten Kraftmessdaten zum Bestimmen von Steuerdaten. Die Steuerdaten sind zur Ansteuerung der Antriebseinheit zur motorischen Unterstützung einer Bewegung der medizintechnischen Komponente bestimmt. Das Verfahren umfasst ferner einen Schritt des Ansteuerns der Antriebseinheit mittels den bestimmten Steuerdaten.

Die erfindungsgemäße Technik kann ein zielgerichtetes und/oder präziseres Verfahren der medizintechnischen Komponente ermöglichen. Alternativ oder ergänzend kann mittels der erfindungsgemäßen Technik eine schnellere, gleichmäßigere und/oder ruckfreiere Fahrt der medizintechnischen Komponente ermöglicht werden. Dadurch kann ein medizinischer Behandlungsablauf oder medizinischer Diagnoseablauf optimiert werden. Beispielsweise kann eine Zeitdauer zwischen der Entscheidung, ein medizinisches Bild aufzunehmen, und der Aufnahme minimiert werden. Alternativ oder ergänzend kann der medizinische Behandlungsablauf oder medizinische Diagnoseablauf dadurch ermöglicht werden, dass die Antriebseinheit zur Bewegung der medizintechnischen Komponente steuerbar ist, insbesondere variabel und/oder situationsspezifisch steuerbar ist, z.B. für einen unerfahrenen, schwachen und/oder leichten Bediener (auch: Benutzer) anders angesteuert werden kann als für einen erfahrenen und/oder starken Bediener. Weiterhin alternativ oder ergänzend kann die Ansteuerung energie-optimiert werden und/oder verschleiß-minimierend (und/oder Verschleiß vermeidend) geplant werden mittels der erfindungsgemäßen Technik. Z.B. kann durch eine sanfte Bewegung mit geringen Änderungen in der Geschwindigkeit und/oder einer geringen Anzahl (und/oder einer geringen Stärke von) Rucks ein Verschleiß der motorischen Unterstützung und/oder der Räder (auch: Rollen) minimiert werden. Alternativ oder ergänzend kann eine sanfte Bewegung die Sicherheit beim Transport eines Patienten erhöhen.

Die Bewegung kann translatorische Freiheitsgrade (und/oder translatorische Bewegungen) und/ oder rotatorische Freiheitsgrade (und/oder rotatorische Bewegungen) umfassen.

Die Bewegung (auch: Fortbewegung) kann eine Bewegung auf einer Ebene und/oder einem Boden, insbesondere auf einer Bodenfläche einer medizinischen Einrichtung (z.B. eines Krankenhauses, einer Fachabteilung, eines Ärztehauses und/oder Arztpraxis), sein.

Die medizintechnische Komponente kann eine bewegliche externe oder eine bewegliche integrierte Komponente eines medizintechnischen, insbesondere bildgebenden, Gerätes sein. Die medizintechnische Komponente kann z.B. ein mobiler Tisch (auch: Patientenliege) zur Lagerung eines Patienten für ein bildgebendes Gerät sein, beispielsweise für einen Magnetresonanztomographen (MRT). Alternativ oder ergänzend kann die medizintechnische Komponente ein mobiler C-Arm sein. Alternativ oder ergänzend kann die medizintechnische Komponente ein mobiles bildgebendes Gerät, beispielsweise ein Computertomograph (CT), sein.

Alternativ oder ergänzend kann die medizintechnische Komponente ein schweres und/oder schwierig manuell anzutreibendes Großgerät sein.

Zur Bewegung kann die medizintechnische Komponente eine Mehrzahl Räder (auch: Rollen) umfassen, die an einer oder mehreren Achsen angeordnet sein können.

Die Bewegung kann eine Längsbewegung und/oder eine Querbewegung umfassen. Die Bewegung kann insbesondere zumindest eine Längsbewegung, und optional eine Querbewegung, umfassen.

Die Längsbewegung kann eine Vorwärtsbewegung und/oder eine Rückwärtsbewegung umfassen. Alternativ oder ergänzend kann die Querbewegung eine Richtungsänderung und/oder eine Rotation (insbesondere um senkrechte Achse) der medizintechnischen Komponente umfassen.

Die Antriebseinheit kann mindestens einen (z.B. elektrischer) Motor umfassen. Z.B. kann die Antriebseinheit einen Motor je Rad (und/oder Rolle) an zumindest einer Radachse (kurz: Achse) umfassen.

Das Bedienelement kann zur manuellen, motorisch unterstützten Bewegung der medizintechnischen Komponente ausgebildet sein. Das Bedienelement kann ein Handgriff und/oder ein Fußbetätigtes Bedienelement (z.B. ein Fußschalter) sein. Das Bedienelement kann als Kraftgriff ausgebildet sein. In der Mechatronik wird ein "Kraftgriff" als eine Art von Handgriff oder Greifmechanismus definiert, der speziell dafür ausgelegt ist, Kräfte zu messen und/oder optional in verarbeiteter Form an andere Komponenten zu übertragen und insbesondere als Steuerdaten zum Ansteuern einer Antriebseinheit an die Antriebseinheit zu übertragen.

Ein Kraftgriff kann als ein Bedienelement definiert werden, z.B. in Form eines Handgriffes oder Schalters oder Mechanismus', das so konstruiert ist, dass es Kräfte, die z.B. von der Hand oder dem Fuß aufgebracht werden, effizient messen, weiterverarbeiten und/oder übertragen kann. Mechanisch besteht der Kraftgriff aus robusten Materialien und kann verschiedene Formen und Grö-ßen haben, um sich an die spezifischen Anforderungen der Anwendung anzupassen. Ein Kraftgriff kann mit Kraftsensoren ausgestattet sein, die die (z.B. manuell) aufgebrachte Kraft messen. Diese Sensoren können Dehnungsmessstreifen, piezoelektrische Sensoren oder andere Arten von Kraftsensoren sein. Die gemessenen Daten werden in Form von Kraftmessdaten gesammelt und können verwendet werden, um Rückmeldungen an ein elektronisches und/oder digitales Steuerungssystem für die Antriebseinheit, wie z.B. an einen Controller, und optional an den Benutzer zu geben. Ein Kraftgriff kann auch als ein elektronisches Gerät definiert werden, das über eine Schnittstelle zur Datenverarbeitung verfügt. Der Kraftgriff kann in ein größeres mechatronisches System integriert sein, in dem die gemessenen Kräfte zur Steuerung von Bewegungen der Antriebseinheit für die medizintechnische Komponente und/oder zur Bereitstellung von Feedback verwendet werden. Die Ansteuerung der Antriebseinheit kann auf Basis der erfassten Kraftmessdaten ausgeführt werden. Insbesondere kann die Antriebskraft proportional zu den Kraftmessdaten bestimmt und/oder angewendet werden. Ein Kraftgriff kann als ein mechatronisches Bedienelement ausgebildet sein, bei dem die durch den Bediener ausgeübte und auf das Bedienelement, insbesondere auf den Kraftgriff, einwirkende Kraft sensorisch in Form von Kraftmessdaten gemessen wird. Beispielsweise kann die Antriebseinheit, z.B. ein Motor, umso stärker angetrieben werden, desto mehr manuelle Kraft auf das Bedienelement ausgeübt wird. Die Steuerdaten können insbesondere proportional zu den erfassten Kraftmessdaten sein. Beispielsweise kann die Antriebseinheit allerdings auch mit geringerer Kraft angetrieben werden, wenn aufgrund anderer Messdaten (z.B. Umgebungsdaten) eine abgebremste Bewegung sinnvoll ist. In einem weiteren Ausführungsbeispiel können die erfassten Kraftmessdaten einer Fläche entsprechen, welche kontaktiert (und/oder berührt) wird. Beispielsweise kann ein Kontakt nur mit Fingerspitzen einem geringen Kraftaufwand entsprechen und ein Kontakt mit der gesamten Handfläche einem hohen Kraftaufwand.

Der Kraftgriff kann ausgebildet sein, eine intendierte Bewegungsrichtung zu erfassen. Der Controller kann ausgebildet sein, die erfassten intendierte Bewegungsrichtung bei der Ansteuerung der Antriebseinheit derart umzusetzen, dass die medizintechnische Komponente in der erfassten intendierten Bewegungsrichtung bewegt wird.

Die medizintechnische Komponente kann einen Motor zur Bewegung oder Bewegungsunterstützung umfassen. Der Motor kann ansteuerbar sein. Die medizintechnische Komponente kann z.B. den Kraftgriff (insbesondere als zumindest einen Teil des Bedienelementes, insbesondere Handgriffs) umfassen.

In einem Ausführungsbeispiel kann die Antriebseinheit zur motorischen Unterstützung (z.B. lediglich) eine Längsbewegung der medizintechnischen Komponente unterstützen. In diesem Ausführungsbeispiel kann eine Richtungsänderung beispielsweise lediglich manuell ausgeführt werden.

In einem weiteren Ausführungsbeispiel kann die Antriebseinheit zur motorischen Unterstützung eine Längsbewegung und eine Richtungsänderung unterstützen. Beispielsweise kann die medizintechnische Komponente mindestens zwei Achsen umfassen. Mindestens eine der Achsen kann eine (insbesondere motorgestützte) Rotationsbewegung ermöglichen.

Das Bestimmen der Steuerdaten kann zumindest teilweise mittels des NN ausgeführt werden. Die Antriebseinheit der motorischen Unterstützung (kurz auch: die motorische Unterstützung) kann eine Aktorik (z.B. umfassend mindestens einen Aktor) umfassen. Die Aktorik kann zumindest einen Motor umfassen.

Die Steuerdaten können zur Ansteuerung der Antriebseinheit, insbesondere der Aktorik, dienen. Die Steuerdaten können alternativ oder ergänzend mehrere Steuerparameter umfassen. Die Steuerparameter können insbesondere ausgewählt sein aus der Gruppe bestehend aus: einer Drehzahl, einer Drehrichtung und/oder einem Drehmoment. Falls die Aktorik mehrere Motoren umfasst, kann jeweils für jeden Motor ein Satz von spezifischen Steuerdaten bestimmt werden, die sich von Motor zu Motor unterscheiden können. Damit entsteht der technische Vorteil, dass die Bewegungssteuerung präziser und genauer ausgeführt werden kann. Z.B. kann dadurch eine Änderung einer Bewegungsrichtung optimiert werden.

Das Verfahren kann in einer elektronischen Einheit (z.B. einem Controller) oder verteilt auf mehrere elektronische Einheiten ausgeführt werden. Insbesondere kann das NN in der verteilten Ausführung auf einem Server implementiert sein, während alle anderen Verfahrensschritte auf einer lokalen Komponente ausgeführt werden. So kann der Schritt des Verarbeitens der Kraftmessdaten und das Bestimmen der Steuerdaten auf dem Server ausgebildet sein. Die bestimmten Steuerdaten werden in diesem Fall an die lokale Komponente (z.B. Controller) gesendet und dort zur Ansteuerung der Antriebseinheit umgesetzt und angewendet.

Die Kraftmessdaten können von einem Kraftmesssensorsystem erfasst werden. Das Kraftmesssensorsystem kann an dem Bedienelement angeordnet sein und zumindest einen Sensor umfassen. Der zumindest eine Sensor kann ein Kraftsensor sein. Alternativ oder ergänzend kann der zumindest eine Sensor ein Berührungssensor sein. Weiterhin alternativ oder ergänzend kann der zumindest eine Sensor ein Richtungssensor sein.

Der Kraftsensor kann dazu ausgebildet sein, eine Kraft zu messen, die auf das Bedienelement (und/oder den Kraftgriff) aufgebracht wird bzw. einwirkt. Damit kann die Intensität des Bewegungswunsches (z.B. hinsichtlich einer Geschwindigkeit und/oder Änderung der Bewegungsrichtung) des Bedieners erfasst werden.

Der Berührungssensor (auch: Kontaktsensor) kann dazu ausgebildet sein, eine Berührung des Bedienelements (und/oder Kraftgriffs) zu detektieren.

Der Richtungssensor kann dazu ausgebildet sein, eine Bewegungsrichtung zur Bewegung der medizintechnischen Komponente zu detektieren.

Das Kraftmesssensorsystem kann mindestens zwei Sensoren umfassen, beispielsweise je einen Sensor links und rechts am Bedienelement, z.B. am Handgriff.

Mittels des Kraftmesssensorsystems kann eine gewünschte Bewegung (z.B. Geschwindigkeit) und/oder Bewegungsänderung (z.B. Beschleunigung und/oder Richtungsänderung), Richtung, Intensität und/oder Stärke (insbesondere einer Kraft auf das Bedienelement, insbesondere den Handgriff) detektiert werden.

Die medizintechnische Komponente kann eine Patientenliege sein, insbesondere für ein bildgebendes Gerät (z.B. für einen MRT). Alternativ oder ergänzend kann die medizintechnische Komponente ein bildgebendes Gerät, wie z.B. ein Röntgengerät sein. Alternativ oder ergänzend kann die medizintechnische Komponente ein CT, insbesondere zur Erfassung eines Kopf-CT, sein. Weiterhin alternativ oder ergänzend kann die medizintechnische Komponente ein C-Arm sein.

Die Patientenliege (auch: Patiententisch; kurz: Tisch) kann zum (zumindest teilweisen) Einfahren eines Patienten in das bildgebende Gerät (z.B. in eine Röhre eines MRT) ausgebildet sein. Die Patientenliege kann z.B. mindestens vier Räder umfassen, insbesondere mit mindestens zwei Rädern an einer Kopfseite und mit mindestens zwei weiteren Rädern an der Fußseite der Patientenliege. In einem Ausführungsbeispiel können die Räder für eine Bewegung in Längsrichtung der Patientenliege fixiert sein. In einem weiteren Ausführungsbeispiel kann zumindest ein Teil der Räder drehbar sein oder an einer drehbaren Achsen montiert sein.

Das Röntgengerät kann ein Komplettgerät zum Einsatz bei Hausbesuchen oder Außeneinsätzen, z.B. in Krisensituationen, umfassen.

Der CT kann in mindestens eine Richtung verfahrbar sein, beispielsweise mittels mindestens vier Rädern. Der mobile CT kann beispielsweise für Kopfaufnahmen geeignet sein. Beispielsweise können CT-Kopfaufnahmen im Verlauf einer Operation (OP) im Kopfbereich (z.B. einer Gehirn-OP) mittels des mobilen CT gemacht werden.

Der C-Arm kann eine Quelle und/oder einen Detektor eines bildgebenden Geräts, beispielsweise eines Röntgengeräts, umfassen. Der C-Arm kann auf einem Fußboden verfahrbar sein, z.B. mit mindestens drei Rädern.

Das Verfahren kann ferner einen Schritt des Empfangens von Umgebungsdaten hinsichtlich einer Umgebung der medizintechnischen Komponente mittels eines Umgebungssensorsystems umfassen. Ein Verarbeiten der Umgebungsdaten und das Bestimmen der Steuerdaten kann ferner auf den empfangenen Umgebungsdaten basieren.

Die Umgebungsdaten können ein Vorhandensein und/oder eine Position von (z.B. mobilen) Hindernissen umfassen. Ein mobiles Hindernis kann z.B. medizinisches Personal, ein Patient und/oder ein anderer Mensch sein. Alternativ oder ergänzend kann das mobile Hindernis eine weitere medizintechnische Komponente, ein Papierkorb, ein Tisch und/oder ein beliebiger Gegenstand (insbesondere ein Gegenstand, der üblicherweise in einer medizinischen Einrichtung vorhanden ist) sein. Beispielsweise kann bei einer Patientenliege für ein bildgebendes Gerät (z.B. ein MRT) der gesamte Aufbau um die Röhre ein Hindernis sein.

Die Umgebungsdaten können alternativ oder ergänzend Wände, Türen und/oder andere Baukomponenten der medizinischen Einrichtung umfassen.

Mittels der empfangenen Umgebungsdaten können Kollisionen der medizintechnischen Komponenten mit Hindernissen, Türen, Wänden, Gegenständen, Menschen und/oder Baukomponenten der medizinischen Einrichtung vermieden werden.

Das Umgebungssensorsystem kann mindestens einen Sensor umfassen. Der mindestens eine Sensor kann ein Radar-Sensor, ein Lidar-Sensor, ein Laser-Sensor, eine Kamera (insbesondere eine dreidimensionalen, 3D, Kamera), einen Beacon und/oder ein Funksignalsensor sein.

Der mindestens eine Umgebungssensor (auch: Lokalisierungssensor und/oder Sensor des Umgebungssensorsystems) kann an der medizintechnischen Komponente angeordnet sein. Beispielsweise ein Radar-Sensor, ein Lidar-Sensor und/oder ein Laser-Sensor kann dazu ausgebildet sein, Reflexionen von einer an der medizintechnischen Komponente angeordneten Quelle ausgesendeten Strahlen zu detektieren. Anhand der detektierten reflektierten Strahlen (kurz: Echo; z.B. anhand einer Einfallsrichtung und/oder einer Zeitverzögerung) kann beispielsweise ein Abstand zu einem Hindernis, einer Wand, einer Tür und/oder einem Menschen bestimmt werden.

Alternativ oder ergänzend kann der mindestens eine Umgebungssensor außerhalb der medizintechnischen Komponente angeordnet sein, beispielsweise an einer weiteren medizintechnischen Komponente und/oder (z.B. fest installiert) in der medizinischen Einrichtung. Z.B. kann der Funksignalsensor an der medizintechnischen Komponente angeordnet sein und Umgebungsdaten von einer außerhalb der medizintechnischen Komponente angeordneten Einheit (z.B. einer Kamera und/oder einer Steuerung einer, z.B. automatischen, Türöffnung) empfangen.

Der Funksignalsensor kann eine Ultra-Wideband (UWB)-Funktechnologie, Bluetooth und/oder WLAN verwenden.

Mittels der Umgebungsdaten kann eine Position der medizintechnischen Komponente bestimmt werden. Alternativ oder ergänzend kann mittels der Umgebungsdaten eine Umgebung kartiert werden und/oder mobile Hindernisse detektiert werden. Hierdurch kann ein Planen einer kollisionsfreien Bewegung der medizintechnischen Komponente ermöglicht werden.

Das Bestimmen der Steuerdaten kann ferner auf einer gespeicherten Umgebungskarte basieren.

Die Umgebungskarte kann lokal in einem Speicher der medizintechnischen Komponente gespeichert sein. Alternativ oder ergänzend kann die Umgebungskarte in einer Cloud gespeichert sein.

Alternativ oder ergänzend kann mittels des Umgebungssensorsystems eine Simultane Positionsbestimmung und Kartierung (fachsprachlich: Simultaneous Localization and Mapping, SLAM) ausgeführt werden.

Mittels der Umgebungskarte kann eine Bewegung der medizintechnischen Komponente relativ zu einem Grundriss der medizinischen Einrichtung geplant werden.

Der Schritt des Bestimmens von Steuerdaten kann zumindest teilweise von dem NN ausgeführt werden.

Das NN kann dazu ausgebildet sein, basierend auf den empfangenen Sensordaten (insbesondere den Kraftmessdaten und optional den Umgebungsdaten) die Bewegung zu planen und/oder Steuerdaten zu bestimmen.

Das NN kann mittels maschinellen Lernens (ML) trainiert sein. Insbesondere kann das NN mittels bestärkenden Lernens (fachsprachlich: Reinforcement Learning, RL) trainiert sein.

Alternativ oder ergänzend kann das NN mit tiefem Lernen (fachsprachlich: Deep Learning, DL) und/oder tiefem bestärkenden Lernen (fachsprachlich: Deep Reinforcement Learning, DRL) trainiert sein. Es können insbesondere Deep-Q-Learning Methoden, Policy-Gradient Methoden und/oder Actor-Critic-Methoden zum Einsatz kommen.

Bei Verwendung von RL lernt ein Agent durch Interaktionen mit seiner Umgebung Aktionen auszuführen, um eine (z.B. kumulative) Belohnung zu maximieren. Die Belohnung (fachsprachlich: reward) kann als Belohnungsfunktion repräsentiert sein und/oder auf Rückmeldungen aus der Umgebung basieren, die den Agenten informieren, wie gut seine Aktionen in Bezug auf das Ziel sind. Die Belohnungsfunktion ist beim klassischen RL bekannt. Alternativ oder ergänzend kann ein inverses bestärkendes Lernen (Inverse Reinforcement Learning, IRL) zur Anwendung kommen, bei dem die Belohnungsfunktion aus Beobachtungen des Verhaltens (z.B. einer Bewegungssteuerung und/oder Bedienung der medizintechnischen Komponente) eines Experten abgeleitet wird.

Das Ziel des Lernprozesses bei Verwendung von RL ist es, eine optimale Policy zu finden, die die erwartete (z.B. kumulative) Belohnung maximiert.

Die Policy (auch: Strategie), um zu bestimmen, welche Aktion der Agent in einem bestimmten Zustand wählen soll, kann über eine Benutzereingabe (z.B. auf einem Bedienelement einer Mensch-Maschine-Schnittstelle) bestimmt und/oder geändert werden.

Die Belohnungsfunktion des Belohnungssystems dient als Feedback-Mechanismus. Die Belohnungsfunktion hilft dem Agenten zu verstehen, welche Aktionen vorteilhaft sind und welche vermieden werden sollten.

Als Strategien für die Belohnungsgestaltung können dichte vs. spärliche Belohnungen verwendet werden. Dichte Belohnungen sind häufige Belohnungen, die dem Agenten kontinuierliches Feedback geben, während spärliche Belohnungen seltene Belohnungen sind, die nur bei wichtigen oder seltenen Ereignissen gegeben werden. Alternativ oder ergänzend können Shaping Rewards zur Anwendung kommen, wobei zusätzliche Belohnungen vergeben werden, um Zwischenschritte zum Ziel zu fördern. Dies hilft, den Lernprozess zu beschleunigen, indem es den Agenten in die richtige Richtung lenkt. Alternativ oder ergänzend können negative Belohnungen (fachsprachlich: Penalties) angewendet werden als Strafen für unerwünschte Aktionen oder Zustände. Die negativen Belohnungen verhindern schädliches und/oder ineffizientes Verhalten. Alternativ oder ergänzend können Reward Scaling Strategien zu Anwendung kommen mit einer Anpassung der Belohnungsskala, um numerische Stabilität und bessere Lernleistung zu gewährleisten. Alternativ oder ergänzend kann Hindsight Experience Replay (HER) als Technik verwendet werden, bei der der Agent aus früheren Erfahrungen lernt, indem er sich vorstellt, dass alternative Ziele erreicht wurden. Dies kann die Effizienz des Lernens in Umgebungen mit spärlichen Belohnungen verbessern.

Alternativ oder ergänzend kann die Strategie dazu dienen, eine Qualität einer Bewegung (insbesondere umfassend eine manuelle Bewegungssteuerung, z.B. durch medizinisches Personal) und/oder einer motorischen Unterstützung zu bewerten. Weiterhin alternativ oder ergänzend können mittels der Strategie die Steuerdaten für die Ansteuerung der Antriebseinheit der motorischen Unterstützung bestimmt werden.

Alternativ oder ergänzend kann die Belohnung manuell (z.B. durch eine Benutzereingabe seitens eines Bedieners mittels einer Benutzerschnittstelle, UI) und/oder automatisch bestimmt werden.

Während des Lernprozesses interagiert der Agent mit der Umgebung basierend auf seiner aktuellen Policy. Für jede Aktion erhält der Agent eine Belohnung, die durch das Belohnungssystem definiert wird. Der Agent verwendet die erhaltenen Belohnungen, um seine Policy zu aktualisieren. Dies kann z.B. durch verschiedene Algorithmen, wie Q-Learning, Policy-Gradient-Methoden oder Actor-Critic-Methoden erfolgen. Grundsätzlich strebt der Agent danach, die Policy so zu verbessern, dass die langfristige (z.B. kumulative) Belohnung maximiert wird, was bedeutet, dass er immer besser darin wird, die optimalen Aktionen in den jeweiligen Zuständen zu wählen.

Zum Trainieren des NN kann ein Belohnungssystem verwendet werden. Das Belohnungssystem kann mindestens eine Eigenschaft einer Bewegung belohnen. Die mindestens eine Eigenschaft der Bewegung kann eine sanfte Bewegung, ein Einhalten eines vorbestimmten Weges, ein Einhalten einer Zeitvorgabe, ein Einhalten einer vorgegebenen Lautstärke und/oder eine Kollisionsfreiheit sein.

In einem Ausführungsbeispiel kann das Trainieren des NN ein Empfangen von Bewertungen eines Bedieners einer ausgeführten Bewegung umfassen. Die Bewertungen können mittels einer Benutzerschnittstelle (fachsprachlich: User Interface, Ul) empfangen werden, beispielsweise mittels einer graphischen Benutzerschnittstelle (GUI).

Der Schritt des Bestimmens von Steuerdaten kann zumindest teilweise von einem (z.B. im Wesentlichen herkömmlichen, vorbekannten und/oder klassischen) Bewegungssteuerungsalgorithmus ausgeführt werden. Der Bewegungssteuerungsalgorithmus kann eine vorbestimmte Parametrisierung der Bewegung in Abhängigkeit der empfangenen Kraftmessdaten umfassen.

Der (insbesondere klassische und/oder vorbekannte) Bewegungssteuerungsalgorithmus kann eine vorbestimmte Parametrisierung der Bewegung umfassen, insbesondere mit einer vorbestimmten (z.B. linearen) Verstärkung von erfassten Kraftmessdaten hinsichtlich einer Kraft auf das Bedienelement, z.B. den Handgriff. Alternativ oder ergänzend kann die vorbestimmte Parametrisierung eine modell-basierte Admittanzregelung umfassen. Die modell-basierte Admittanzregelung kann z.B. vorbestimmte Toleranzen und/oder Abweichungen hinsichtlich einer maximalen (z.B. positiven und/oder negativen) Beschleunigung, einer maximalen Richtungsänderung pro Zeit und/oder eines minimalen Abstands zu einem Hindernis umfassen.

Der Bewegungssteuerungsalgorithmus kann in einem Ausführungsbeispiel eine Konfiguration der vorbestimmten Parametrisierung umfassen, die Bediener-spezifisch ist, optional mit manueller Konfiguration durch den Bediener und/oder automatischer Erkennung des Bedieners. Z.B. kann ein Bediener, der zum medizinischen Personal der medizinischen Einrichtung gehört, anhand einer individuellen Kennung (z.B. mittels eines Transponders und/oder eines Mitarbeiterausweises) erkannt werden. Alternativ oder ergänzend kann ein Bediener aus einer vordefinierten Gruppe von Bedienern z.B. anhand von Gesichtserkennung, Iriserkennung und/oder Fingerabdruckabgleich erkannt werden.

In einem weiteren Ausführungsbeispiel kann die vorbestimmte Parametrisierung des Bewegungssteuerungsalgorithmus lernend angepasst werden, z.B. anhand von Ausgaben des NN. Beispielsweise kann sich ein Bewegungsprofil der medizintechnischen Komponente über dessen Lebenszeit (z.B. aufgrund von Verschleißerscheinungen) verändern. Die lernende Anpassung der vorbestimmten Parametrisierung kann die Veränderung des Bewegungsprofils berücksichtigen. Dadurch kann eine (z.B. minimale und/oder durchschnittliche) Qualität der Ansteuerung der Antriebseinheit während der Lebensdauer der medizintechnischen Komponente garantiert werden.

In einem Ausführungsbeispiel kann das Bestimmen der Steuerdaten ein Fusionieren einer Ausgabe des NN mit dem Bewegungssteuerungsalgorithmus umfassen. Beispielsweise können die Ausgaben des NN und des Bewegungssteuerungsalgorithmus (z.B. gewichtet) addiert und/oder gemittelt werden. Das gewichtete Addieren und/oder die gewichtete Mittelung kann z.B. den Anteil der mittels des NN verarbeiteten Kraftmessdaten auf einen Maximalanteil (z.B. 10%) begrenzen. Dadurch kann eine Feinabstimmung (fachsprachlich: Fine Tuning) des Bewegungssteuerungsalgorithmus stattfinden, wobei gleichzeitig unvorhergesehene und/oder unkontrollierte Bewegungsabläufe verhindert werden. Alternativ oder ergänzend kann der Bewegungssteuerungsalgorithmus zur Validierung und/oder Plausibilitätsprüfung der Ausgabe des NN anwendbar sein.

Hierdurch kann eine funktionale Sicherheit der medizintechnischen Komponente erhöht werden. Alternativ oder ergänzend können schwerwiegende Fehler des NN abgemildert werden.

Das NN kann anhand von simulierten Bewegungsdaten der medizintechnischen Komponente trainiert werden. Alternativ oder ergänzend kann das NN anhand von historischen Bewegungsdaten der medizintechnischen Komponente trainiert werden.

Das NN kann als Trainingsdaten simulierte Bewegungsdaten (z.B. eines digitalen Zwillings) und/oder historische Bewegungsdaten der medizintechnischen Komponente (und/oder einer baugleichen Kopie der medizintechnischen Komponente) empfangen zusammen mit einer Bewertung entsprechend dem Belohnungssystem. Als Ground Truth können die Steuerdaten der simulierten und/oder historischen Bewegungsdaten in einem Trainingsdatensatz enthalten sein.

In einem Ausführungsbeispiel können zwei zumindest im wesentlichen baugleiche medizintechnische Komponenten gemeinsam trainiert werden.

Ein Vor-Training kann während der Entwicklung und/oder der Produktion der medizintechnischen Komponente erfolgen, beispielsweise mittels des digitalen Zwillings der medizintechnischen Komponente.

Das NN kann während der Lebensdauer der medizintechnischen Komponente nachtrainiert werden. Dadurch können beispielsweise Änderungen von physikalischen Eigenschaften (z.B. aufgrund von Verschleiß) während der Lebensdauer der medizintechnischen Komponente berücksichtigt werden.

Das Vor-Training und/oder Training des NN kann auf die Einbausituation und/oder den bestimmungsgemäßen Verwendungsort (auch: Einsatzort) der medizintechnischen Komponente ausgelegt sein. Beispielsweise kann beim Vor-Training und/oder Training des NN ein Lageplan und/oder Grundriss (z.B. als Umgebungsplan) eines Bereichs bzw. einer Abteilung (z.B. eines OP-Bereichs und/oder einer Bildgebungs-Abteilung) einer medizinischen Einrichtung berücksichtigt werden.

Das Verfahren kann zumindest teilweise (wie oben bereits beschrieben, kann das Verfahren auch auf verteilten Einheiten ausgeführt werden) von einem Controller ausgeführt werden. Vorzugsweise ist der Controller zur Ausführung des Verfahrens ausgebildet. Der Controller kann an der medizintechnischen Komponente angeordnet sein (fachsprachlich: Edge Device). Alternativ oder ergänzend kann der Controller (z.B. zentral) in der medizinischen Einrichtung angeordnet sein (fachsprachlich: On-Premise Device). Der Controller kann zur Steuerung zumindest einer medizintechnischen Komponente ausgebildet sein.

Das Verfahren kann in einem Ausführungsbeispiel lokal auf der medizintechnischen Komponente ausgeführt werden (z.B. nach anfänglichem Training des NN außerhalb und Übertragung des trainierten NN auf die medizintechnische Komponente).

In einem weiteren Ausführungsbeispiel kann das Verfahren Cloud-basiert sein. Beispielsweise kann eine an der medizintechnischen Komponente lokalisierte Aktorik über eine drahtlose Datenverbindung mit einem NN und/oder einem Bewegungssteuerungsalgorithmus verbindbar sein, um die Steuerdaten zu empfangen. Alternativ oder ergänzend kann das Umgebungssensorsystem zumindest teilweise außerhalb der medizintechnischen Komponente angeordnet sein.

In einem Ausführungsbeispiel kann das Vor-Training des Controllers zentral und/oder Cloud-basiert ausgeführt werden. Alternativ oder ergänzend kann das Training des Controllers während der Lebensdauer der medizintechnischen Komponente (insbesondere unabhängig von einem Vor-Training) lokal auf der medizintechnischen Komponente und/oder Cloud-basiert ausgeführt werden.

Ein Cloud-basiertes Vor-Training ermöglicht vorteilhafterweise eine schlanke Hardware (und/oder einen kleinen Controller), auf die das vor-trainierte NN übertragen wird (z.B. lokal auf die medizintechnische Komponente).

Die Ansteuerung der Antriebseinheit zur motorischen Unterstützung kann deaktivierbar sein. Die Deaktivierung kann z.B. manuell an einer Benutzerschnittstelle (UI), insbesondere einer graphischen Benutzerschnittstelle (fachsprachlich: Graphical User Interface, GUI) erfolgen.

In einem Ausführungsbeispiel kann die Deaktivierung lediglich bewirken, dass die bestimmten Steuerdaten nicht auf die Antriebseinheit angewendet und/oder nicht an die Antriebseinheit gesendet werden. In einem weiteren Ausführungsbeispiel kann die Deaktivierung bewirken, dass die Schritte des Verarbeitens der Kraftmessdaten und/oder des Bestimmens der Steuerdaten nicht ausgeführt werden.

Das Verfahren kann ferner einen Schritt des Ausgebens eines Signals hinsichtlich einer Qualität der Bewegungssteuerung durch die erfassten Kraftmessdaten umfassen. Die Qualität kann im Schritt des Verarbeitens der Kraftmessdaten durch das NN ermittelt werden.

Die Qualität der Bewegungssteuerung durch die erfassten Kraftmessdaten kann eine Korrelation zwischen einer gemessenen Kraft eines Bedieners und einer Qualität der Bewegung (z.B. anhand des Reward-Systems) umfassen. Dadurch kann ein Bediener dazu veranlasst werden, die Kraft auf das Bedienelement, wie z.B. auf den Handgriff geeignet zu dosieren. Dadurch kann ein Verschleiß am Bedienelement minimiert werden, beispielsweise durch ein Vermeiden von zu gro-ßen Kraftaufwendungen.

Das Signal kann an einer Benutzerschnittstelle, insbesondere einem GUI, ausgegeben werden. Alternativ oder ergänzend kann das Signal gemäß einer Ampel ausgegeben werden (z.B. "grün" für "gut", "rot" für "schlecht", und optional "gelb" für "noch akzeptabel).

Gemäß einem Vorrichtungsaspekt wird ein Controller zum Ansteuern einer Antriebseinheit zur motorischen Unterstützung einer Bewegung einer medizintechnischen Komponente bereitgestellt. Der Controller umfasst eine Kraftdatenerfassungseinheit, die zum Erfassen von Kraftmessdaten ausgebildet ist. Die Kraftmessdaten repräsentieren eine Kraft, die zur Bewegungssteuerung einer mobilen motorisch unterstützten medizintechnischen Komponente an einem Bedienelement an der medizintechnischen Komponente aufgebracht wird. Das Bedienelement kann insbesondere ein Handgriff sein. Der Controller umfasst ferner eine Verarbeitungseinheit, die dazu ausgebildet ist, mittels eines NN die erfassten Kraftmessdaten zu verarbeiten. Der Controller umfasst ferner eine Bestimmungseinheit, die zum Bestimmen von Steuerdaten zur Ansteuerung einer Antriebseinheit zur motorischen Unterstützung einer Bewegung der medizintechnischen Komponente ausgebildet ist. Der Controller umfasst ferner eine Ansteuerungsschnittstelle, die dazu ausgebildet ist, die Antriebseinheit mittels den bestimmten Steuerdaten anzusteuern.

Der Controller kann ferner eine Umgebungsdatenempfangseinheit umfassen, die dazu ausgebildet ist, Umgebungsdaten hinsichtlich einer Umgebung der medizintechnischen Komponente mittels eines Umgebungssensorsystems zu empfangen und/oder zu erfassen.

Der Controller kann dazu ausgebildet sein, das Verfahren gemäß dem Verfahrensaspekt auszuführen. Alternativ oder ergänzend kann der Controller eines oder mehrere Merkmale umfassen, die im Rahmen des Verfahrensaspekts offenbart sind.

Gemäß einem Systemaspekt wird ein System zum Ansteuern einer Antriebseinheit zur motorischen Unterstützung einer Bewegung einer medizintechnischen Komponente bereitgestellt. Das System umfasst einen Controller gemäß dem Vorrichtungsaspekt. Das System umfasst ferner mindestens eine medizintechnische Komponente mit mindestens einer Antriebseinheit zur motorischen Unterstützung der Bewegung.

Gemäß einem weiteren Aspekt wird ein Computerprogrammprodukt bereitgestellt mit Programmelementen, die einen Controller veranlassen, die Schritte des Verfahrens zum Ansteuern einer Antriebseinheit zur motorischen Unterstützung einer Bewegung einer medizintechnischen Komponente gemäß dem Verfahrensaspekt auszuführen, wenn die Programmelemente in einen Speicher des Controllers geladen werden.

Gemäß einem noch weiteren Aspekt wird ein computerlesbares Medium bereitgestellt, auf dem Programmelemente gespeichert sind, die von einem Controller gelesen und ausgeführt werden können, um Schritte des Verfahrens zum Ansteuern einer Antriebseinheit zur motorischen Unterstützung einer Bewegung einer medizintechnischen Komponente gemäß dem Verfahrensaspekt durchzuführen, wenn die Programmelemente von dem Controller ausgeführt werden.

Die oben beschriebenen Eigenschaften, Merkmale und Vorteile der vorliegenden Erfindung sowie die Art und Weise, wie sie erreicht werden, werden im Lichte der folgenden Beschreibung und der Ausführungsbeispiele, die im Zusammenhang mit den Zeichnungen näher erläutert werden, klarer und verständlicher. Diese folgende Beschreibung beschränkt die Erfindung nicht auf die enthaltenen Ausführungsformen. Gleiche Komponenten oder Teile können in verschiedenen Figuren mit den gleichen Bezugszeichen versehen sein. Im Allgemeinen sind die Abbildungen nicht maßstabsgetreu.

Es versteht sich, dass eine bevorzugte Ausführungsform der vorliegenden Erfindung auch eine beliebige Kombination der abhängigen Ansprüche oder der obigen Ausführungsformen mit dem jeweiligen unabhängigen Anspruch sein kann.

Diese und andere Aspekte der Erfindung werden aus den nachfolgend beschriebenen Ausführungsformen ersichtlich und werden durch Bezugnahme auf diese erläutert.

### KURZE BESCHREIBUNG DER ZEICHNUNGEN

- Fig. 1: ist ein Flussdiagramm eines Verfahrens zum Ansteuern einer Antriebseinheit zur motorischen Unterstützung einer Bewegung einer medizintechnischen Komponente gemäß einer bevorzugten Ausführungsform der vorliegenden Erfindung.
- Fig. 2: ist eine Übersicht über die Struktur und den Aufbau eines Controllers gemäß einer bevorzugten Ausführungsform der vorliegenden Erfindung.
- Fig. 3: zeigt schematisch eine herkömmliche Ansteuerung einer motorischen Unterstützung eines schweren mobilen Objekts.
- Fig. 4: illustriert schematisch das Grundprinzip des verstärkenden Lernens (RL).
- Fig. 5: illustriert schematisch eine Trainingsphase eines neuronalen Netzwerks, das zum Ausführen von Schritten des Verfahrens der Fig. 1 trainiert wird.
- Figs. 6A und 6B: illustrieren schematisch eine Inferenzphase des gemäß Fig. 5 trainierten und/oder in einem Controller gemäß Fig. 2 integrierten neuronalen Netzwerks.
- Fig. 7: illustriert schematisch Datenverbindungen einer mobilen medizintechnischen Komponente, die mit einem Controller gemäß Fig. 2 ausgestattet ist und/oder die mittels des Verfahrens der Fig. 1 ansteuerbar ist.
- Figs. 8, 9, 10 und 11: zeigen schematisch Ausführungsbeispiele einer mobilen medizintechnischen Komponente (z.B. der medizintechnischen Komponente aus Fig. 7), insbesondere eine zu einem MRT gehörende Patientenliege, einen mobilen CT, einen mobilen C-Bogen bzw. ein mobiles Röntgengerät.

Etwaige Bezugszeichen in den Ansprüchen sind nicht als Einschränkung des Anwendungsbereichs zu verstehen.

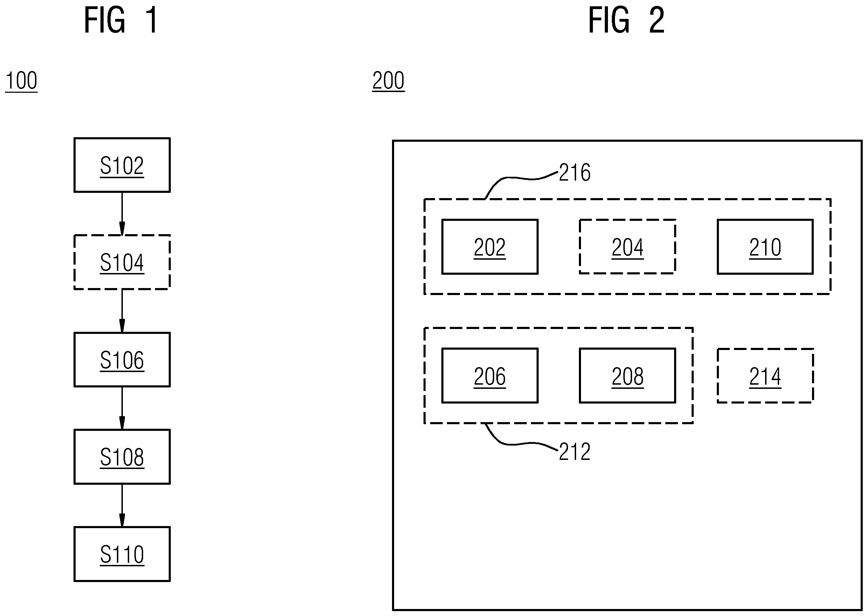

Fig. 1 zeigt schematisch ein beispielhaftes Ablaufdiagramm eines (insbesondere computer-implementierten) Verfahrens 100 zum Ansteuern einer Antriebseinheit zur motorischen Unterstützung einer Bewegung einer medizintechnischen Komponente.

Das Verfahren 100 umfasst einen Schritt S102 des Erfassens von Kraftmessdaten. Die Kraftmessdaten repräsentieren eine Kraft, die zur Bewegungssteuerung einer mobilen motorisch unterstützten medizintechnischen Komponente an einem Bedienelement an der medizintechnischen Komponente aufgebracht wird. Das Bedienelement kann insbesondere ein Handgriff sein.

Das Verfahren 100 umfasst ferner einen Schritt S106 des Verarbeitens, mittels eines neuronalen Netzwerks (NN), der erfassten S102 Kraftmessdaten und einen Schritt S108 des Bestimmens von Steuerdaten zur Ansteuerung der Antriebseinheit zur motorischen Unterstützung einer Bewegung der medizintechnischen Komponente anhand der verarbeiteten S106 Kraftmessdaten.

Das Verfahren 100 umfasst einen Schritt S110 des Ansteuerns der Antriebseinheit mittels den bestimmten S108 Steuerdaten.

Das Verfahren 100 kann einen Schritt S104 des Empfangens von Umgebungsdaten hinsichtlich einer Umgebung der medizintechnischen Komponente umfassen Die Umgebungsdaten können mittels eines Umgebungssensorsystems empfangen S104 werden.

Ein (z.B. gleichzeitiges oder gemeinsames) Verarbeiten S106 der Kraftmessdaten und der Umgebungsdaten und das Bestimmen S108 der Steuerdaten kann ferner auf den empfangenen S104 Umgebungsdaten basieren.

Das Verfahren 100 kann ferner einen Schritt (nicht in Fig. 1 gezeigt) des Ausgebens eines Signals hinsichtlich einer Qualität der Bewegungssteuerung durch die erfassten S102 Kraftmessdaten umfassen. Die Qualität kann beispielsweise im Schritt des Verarbeitens S106 der Kraftmessdaten (und optional der Umgebungsdaten) durch das NN ermittelt werden.

Fig. 2 zeigt schematisch eine Ausführungsbeispiel eines Controllers 200 zum Ansteuern einer Antriebseinheit zur motorischen Unterstützung einer Bewegung einer medizintechnischen Komponente.

Der Controller 200 umfasst eine Kraftdatenerfassungseinheit 202, die zum Erfassen von Kraftmessdaten ausgebildet ist. Die Kraftmessdaten repräsentieren eine Kraft, die zur Bewegungssteuerung einer mobilen motorisch unterstützten medizintechnischen Komponente an einem Bedienelement an der medizintechnischen Komponente aufgebracht wird. Das Bedienelement kann insbesondere ein Handgriff sein.

Der Controller 200 umfasst ferner eine Verarbeitungseinheit 206, die dazu ausgebildet ist, mittels eines NN die erfassten Kraftmessdaten zu verarbeiten.

Der Controller 200 umfasst ferner eine Bestimmungseinheit 208, die zum Bestimmen von Steuerdaten zur Ansteuerung einer Antriebseinheit zur motorischen Unterstützung einer Bewegung der medizintechnischen Komponente ausgebildet ist.

Der Controller 200 umfasst ferner eine Ansteuerungsschnittstelle 210, die dazu ausgebildet ist, die Antriebseinheit mittels den bestimmten Steuerdaten anzusteuern.

Die Controller 200 kann eine Umgebungsdatenempfangseinheit 204 umfassen, die dazu ausgebildet ist, Umgebungsdaten hinsichtlich einer Umgebung der medizintechnischen Komponente zu empfangen (und/oder zu erfassen). Die Umgebungsdaten können mittels eines Umgebungssensorsystems empfangen (und/oder erfasst) werden.

Der Controller 200 kann einen Prozessor 212 umfassen. Beispielsweise können die Verarbeitungseinheit 206 und die Bestimmungseinheit 208 durch den Prozessor 212 realisiert sein.

Der Controller 200 kann eine Speichereinheit (kurz: Speicher; auch: Speichermedium, Medium) 214 umfassen. Beispielsweise können auf der Speichereinheit 214 Programmelemente gespeichert sein, um Schritte des Verfahrens 100 auszuführen.

Der Controller 200 kann eine Eingabe-Ausgabe-Schnittstelle 216 umfassen. Die Kraftdatenerfassungseinheit 202, die optionale Umgebungsdatenempfangseinheit 204 und/oder die Ansteuerungsschnittstelle 210 können beispielsweise in der Eingabe-Ausgabe-Schnittstelle 216 zusammengefasst sein.

Der Controller 200 kann Teil eines Systems zum Ansteuern einer Antriebseinheit zur motorischen Unterstützung einer Bewegung einer medizintechnischen Komponente sein. Das System kann ferner mindestens eine medizintechnische Komponente umfassen mit einer Antriebseinheit zur motorischen Unterstützung der Bewegung.

Der Controller 200 kann lokal an der medizintechnischen Komponente angeordnet sein. In einem alternativen Ausführungsbeispiel können zumindest die Kraftdatenerfassungseinheit 202 und die Ansteuerungsschnittstelle 210 lokal an der medizintechnischen Komponente angeordnet sein. In diesem Ausführungsbeispiel können beispielsweise die Verarbeitungseinheit 206 und die Bestimmungseinheit 208 auf einer zentralen Rechenvorrichtung oder in einer Cloud realisiert sein.

Die erfindungsgemäße Technik (z.B. umfassend das Verfahren 100, den Controller 200 und/oder das System) kann auch als Machine-Learning basierte kraftunterstützte Bewegungsansteuerung von medizinischen Geräten bezeichnet werden.

Medizintechnische Komponenten (bzw. Geräte), bei denen eine kraftunterstützte Bewegungsansteuerung sinnvoll oder teilweise sogar notwendig ist, umfassen z.B. ein Deckenstativ mit einem 3D Kraftsensor und einer Ansteuerung von drei motorischen Achsen.

Für die erfindungsgemäße Technik insbesondere relevant sind ein mobiler Patiententisch (z.B. zugehörig zu einem MRT). Der Patiententisch kann beispielsweise zwei Kraftsensoren an einem Griff (insbesondere Handgriff) umfassen, über welchen mindestens ein motorisches Unterstützungsrad gesteuert wird. Alternativ oder ergänzend kann die erfindungsgemäße Technik bei einem mobilen CT, insbesondere einem Kopf-CT angewendet werden. Der CT kann beispielsweise einen Griff (insbesondere Handgriff) mit zwei Kraftsensoren umfassen, welcher zwei oder mehr Räder antreibt.

Die erfindungsgemäße Technik löst das Problem unter Anwendung von maschinellem Lernen (fachsprachlich: Machine Learning, ML). Ein vielversprechender ML-Ansatz für das Problem ist verstärkendens Lernen (auch: bestärkendes Lernen, fachsprachlich: Reinforcement Learning, RL), insbesondere RL, welches auf neuronale Netzwerke (NNs) zur Generalisierung des Wissens zugreift. Sofern NNs mit mehreren Schichten eingesetzt werden, spricht man von tiefen verstärkendem Lernen (fachsprachlich: Deep RL).

Hiermit ist es möglich, von qualitativ hochwertigen Bedienern die optimale Ansteuerung zu lernen und dieses Wissen (auch: "Policy" in RL) bei unerfahrenen Benutzern anzuwenden und sie bei der Ansteuerung der medizintechnischen Komponente (auch: des medizinischen Geräts) zu unterstützen.

Fig. 4 zeigt schematisch das Grundprinzip des RL. Von einem Agent 404 wird Aktion Aₜ an eine Umgebung 402 übermittelt. Die Umgebung 402 übermittelt einen Zustand St bw. Sₜ₊₁ und eine Belohnung (auch: Reward, Gewinn) Rₜ bzw. Sₜ₊₁ pro Instanz (und/oder Zeitpunkt) t bzw. t+1.

Dem Agent 404 ist im Rahmen der erfindungsgemäßen Technik die medizintechnische Komponente (auch: medizinische Gerät bzw. ein Teil davon) zugeordnet, und Umgebung 402 umfasst die Rückkopplung zwischen der ausgeführten Aktion A der medizintechnischen Komponente (auch: des Geräts) und dem Verhalten des Menschen, welcher die medizintechnische Komponente (auch: das Gerät) bedient. Der Bediener löst hierbei eine Aktion aus (insbesondere wird eine Kraft F an der medizintechnischen Komponente bzw. am Gerät aufgewandt, welche durch ein Kraftmesssensorsystem, kurz: Sensorik, messbar ist) und verändert damit den Zustand S des Systems. Wichtig für das Umsetzen des Lernens ist der Reward R (auch: Gewinn), welcher ausdrückt, wie "gut" die Aktion A war. Ziel des Lernverfahrens ist es, den zukünftig zu erwartenden Gewinn R zu maximieren.

Bei RL wird hierbei ein NN eingesetzt, über welches eine "Policy" (auch: Strategie) erlernt wird. Mit Hilfe der Policy kann später die "beste" Aktion A im gegebenen Zustand S ermittelt werden. Ein NN kann dabei entweder zur Bewertung der gegenwärtigen Policy zum Einsatz kommen (Critic), zur Bestimmung der nächsten Aktion selbst (Actor), oder beides (Actor/Critic).

Falls der Zustand S des Systems nicht beobachtet werden kann, so kann ein "partially observable Markov decision process" (POMDP) angewendet werden. Hier wird der Zustand S durch die Observation O ersetzt.

Im Rahmen der erfindungsgemäßen Technik kann der Zustand S und/oder die Observation O die Krafteinwirkung F sein. Die Aktion A kann die Motoransteuerung sein. Der Reward R kann beispielsweise einer "Sanften Bewegung" entsprechen, welche gemessen und bewertet wird durch Geschwindigkeit, und/oder erste (1.) und/oder zweite (2.) Ableitung der Geschwindigkeit der medizintechnischen Komponente (bzw. des medizinischen Geräts).

Alternative oder ergänzende Rewards können sich beziehen auf Abweichung vom optimalen Weg. Beispielsweise kann ein Einhalten des optimalen Wegs mit dem maximalen Reward bewertet werden. Für das Bestimmen des optimalen Wegs kann eine sensorische Lokalisierung und ein Wissen über das Ziel verwendet werden. Weiterhin alternativ oder ergänzend kann ein Reward sich auf eine Eingabe (auch: Input) vom Benutzer (auch: Bediener) beziehen. Beispielsweise kann ein Benutzer mittels einer Benutzerschnittstelle (z.B. je eine Taste für "gute" oder "schlechte" Bewegung als) eine Bewertung der Bewegung abgeben. Eine gute Bewertung kann einem hohen Reward entsprechen, und eine schlechte Bewertung kann einem niedrigen (oder gar keinem) Reward entsprechen.

Alternativ oder ergänzend kann im Rahmen des RL der Zustand S und/oder die Observation O eine aktuelle Geschwindigkeit der medizintechnischen Komponente (bzw. des Geräts) und/oder eine Krafteinwirkung gemessen an einem Sensor repräsentieren. Die Aktion kann ein (insbesondere additives) Signal auf die Motoransteuerung sein. Der oder die Rewards können wie vorstehend sein und beispielsweise eine Optimierung der Bewegung bewerten.

Alternativ oder ergänzend kann im Rahmen des RL ein Zustand S und/oder eine Observation O durch eine aktuelle Geschwindigkeit der medizintechnischen Komponente (bzw. des Geräts) und/oder eine Krafteinwirkung gemessen am Sensor repräsentiert sein. Die Aktion kann einen oder mehrere gegenwärtig anzuwendende Parameter der Admittanzregelung betreffen (beispielsweise bestimmen oder festlegen). Der oder die Rewards können wie vorstehend sein und beispielsweise eine Optimierung der Bewegung bewerten.

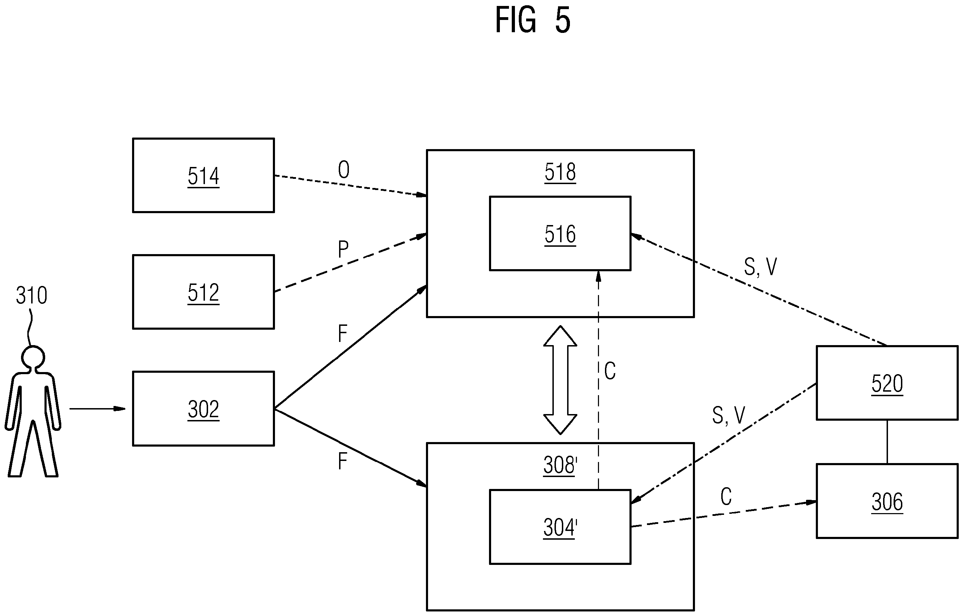

Fig. 5 zeigt ein Beispiel einer Trainingsphase gemäß der erfindungsgemäßen Technik. Ein Kraftsensor 302 (als Beispiel eines Sensors eines Kraftmesssensorsystems) erfasst Kraftmessdaten (kurz: Kraft) F hinsichtlich einer von einem Bediener 310 aufgewendeten Kraft und leitet diese als Eingabe (auch: Input) an ein NN 516 und einen (z.B. im Wesentlichen konventionellen, beispielsweise bis auf eine Anpassbarkeit der Parameter) Bewegungssteuerungsalgorithmus 304`. In der Trainingsphase können das zu trainierende NN 516 und der Bewegungssteuerungsalgorithmus 304` voneinander getrennt angeordnet sein. Beispielsweise kann das NN 516 auf einer zentralen Vorrichtung (auch: Edge-Hardware) 518 oder in einer Cloud trainiert werden. Der Bewegungssteuerungsalgorithmus 304` kann auf einem (z.B. im Wesentlichen konventionellen) Steuerungssystem 308' angeordnet sein.

Wie in Fig. 5 beispielhaft gezeigt kann ein Lokalisierungssensor 512 (beispielsweise als Teil eines Umgebungssensorsystems) Lokalisierungssensordaten bzw. eine daraus abgeleitete absolute Pose P dem NN 516 bereitstellen. Auf Basis der Lokalisierungssensordaten kann die absolute Pose P der medizintechnischen Komponente (bzw. des Geräts) im Raum geschätzt werden. Beispielsweise kann die Pose P Koordinaten des Schwerpunkts der medizintechnischen Komponente (bzw. des Geräts) und/oder eine Orientierung im Raum der medizintechnischen Komponente (bzw. des Geräts) umfassen. Die Pose P kann in einem Ausführungsbeispiel bereits im oder am Lokalisierungssensor 512 bestimmt werden. In einem weiteren Ausführungsbeispiel kann die Pose P im NN 516 anhand der Lokalisierungssensordaten geschätzt werden. Beide Ausführungsbeispiele sind kombinierbar. Beispielsweise kann das NN 516 sowohl die geschätzte Pose P als auch die Lokalisierungssensordaten als Rohdaten empfangen und eine Kontrollabschätzung der Pose P ausführen.

Im Ausführungsbeispiel der Fig. 5 empfängt das NN 516 ferner Daten aus einer Umgebungskarte (kurz: Karte) 514. Insbesondere können die Kartendaten (z.B. Positionen und/oder Abmessungen) bekannte(r) Objekte O umfassen. Mittels der Umgebungskarte 514 können feste Hindernisse (z.B. inklusive Wände und Türen) am Einsatzort der medizintechnischen Komponente (bzw. des Geräts) erlernt werden.

In Fig. 5 sind ferner beispielhaft ein Motor 306 und ein zugeordneter Positionssensor 520 gezeigt. Der Motor 306 (und optional der Positionssensor 520) kann eine Antriebseinheit der medizintechnischen Komponente (bzw. des Geräts) verkörpern.

Der (z.B. im Wesentlichen konventionelle) Bewegungssteuerungsalgorithmus 304` kann in der Trainingsphase der Fig. 5 Steuerdaten (auch: Control Output) C an das zu trainierende NN 516 und an den Motor 306 ausgeben.

Der beispielhafte Positionssensor 520 des Motors kann an den (z.B. im Wesentlichen konventionellen) Bewegungssteuerungsalgorithmus 304` und an das zu trainierende NN 516 in der Trainingsphase Sensordaten hinsichtlich der Position S des Motors 306 (und/oder eines zugeordneten Rads) sowie hinsichtlich der Geschwindigkeit V des Motors 306 (bzw. des zugeordneten Rads) übertragen.

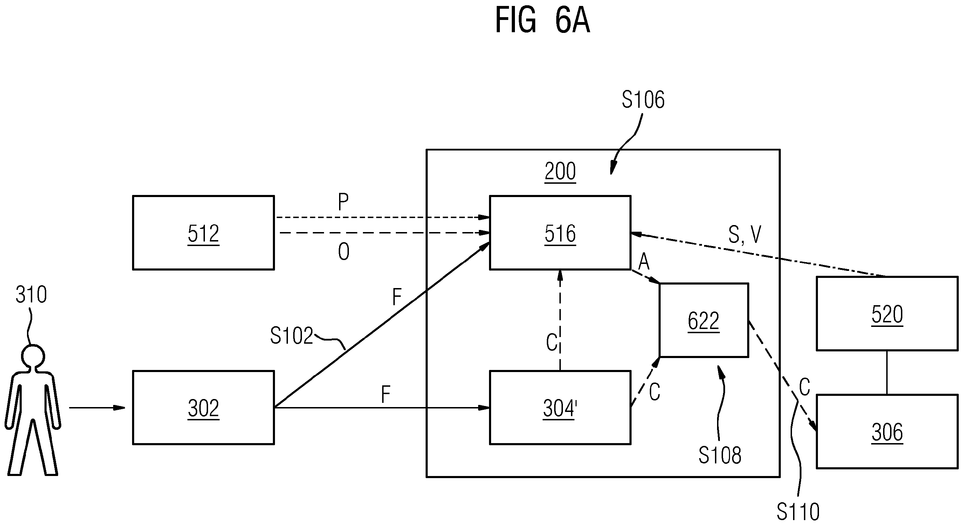

Im Vergleich zu konventionellen Techniken ist die Kraft F, wie beispielhaft in Fig. 5 für die Trainingsphase und in Fig.6A für die Inferenzphase gezeigt, eine Eingabe (auch: Input) für das NN 516. In dem in Fig. 6A gezeigten Ausführungsbeispiel ist die Ausgabe (auch: Output) des NN 516 eine Aktion A, welche zusammen mit der Ansteuerung C aus dem (z.B. im Wesentlichen konventionellen) Bewegungssteuerungsalgorithmus 304` in eine Fusionseinheit (bzw. einen Block "Aktion Fusion") 622 eingeht.

Im Ausführungsbeispiel der Inferenzphase des trainierten NN 516 der Fig. 6A wird der Schritt S106 des Verfahrens 100 des Verarbeitens der erfassten S102 Kraftmessdaten zum Bestimmen von Steuerdaten zur Ansteuerung des Motors 306 (bzw. der Antriebseinheit 306 zur motorischen Unterstützung) im NN 516 ausgeführt. Die Steuerdaten werden in der Fusionseinheit 622 unter Berücksichtigung der Steuerdaten des (z.B. im Wesentlichen konventionellen) Bewegungssteuerungsalgorithmus 304` bestimmt S108. Anhand der Ausgabe der Fusionseinheit 622 wird der Motor 306 angesteuert S110.

Wie in Fig. 6A beispielhaft an Bezugszeichen 622 gezeigt kann die Fusionseinheit (bzw. der Block) die Aktion A des NN 516 auf die Ansteuerung C applizieren des (z.B. im Wesentlichen konventionellen) Bewegungssteuerungsalgorithmus 304`. Hierbei kann eine Validierung und/ oder Plausibilitätsprüfung erfolgen. Die Validierung und/oder Plausibilitätsprüfung kann beispielweise eine (z.B. gewichtete) "Addition" in einem vorgegebenen zulässigen Band sein. Alternativ oder ergänzend sind andere Verfahren zur Validierung und/oder Plausibilitätsprüfung denkbar bzw. anwendbar.

Hauptgrund für die Einführung der Fusionseinheit (bzw. des Blocks "Aktion Fusion") 622 in dem Ausführungsbeispiel der Fig. 6A ist, dass hiermit ein Nachweis bezüglich einer funktionalen Sicherheit des Gesamtsystems leichter als konventionellerweise erbracht werden kann. Beispielsweise kann die Aktion A aus dem NN 516 nur in einem "plausiblen" Bereich appliziert werden. Wenn beispielsweise der (z.B. im Wesentlichen konventionelle) Bewegungssteuerungsalgorithmus 304` aufgrund der Krafteinwirkung F eine Bewegungsführung nach vorne berechnet, kann eine Aktion A nach hinten aus dem NN 516 verboten bzw. nicht erlaubt sein.

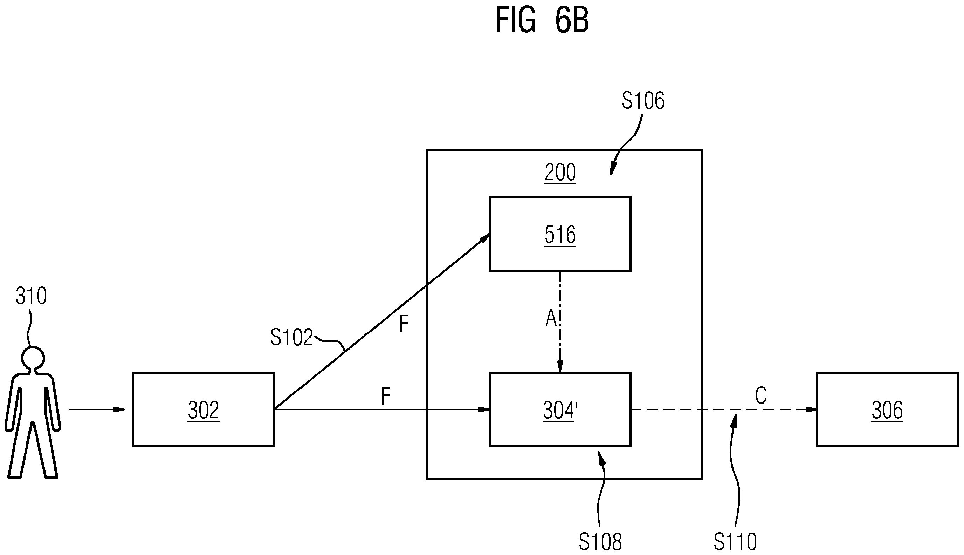

Wie schematisch in Fig. 6B illustriert, kann die Aktion A des NN 516 die Parametrisierung des (z.B. im Wesentlichen konventionellen) Bewegungssteuerungsalgorithmus 304` lernend adaptieren. Andere in Fig. 6A gezeigte Komponenten und/oder Datenflüsse können zusätzlich zu den in Fig. 6B gezeigten Merkmalen vorhanden sein.

Das NN 516 wird in dem Ausführungsbeispiel der Fig. 5 nicht direkt auf dem Controller (bzw. Steuerungssystem) 200 trainiert, sondern auf einer Trainingsvorrichtung (auch: zentrale Rechenvorrichtung und/oder "Edge Hardware", kurz: "Edge HW') 518, welche mehr Ressourcen zur Verfügung hat. Hierzu werden die notwendigen Größen Zustand S und/oder Observation O (kurz: S/O), Aktion A, Steuersignal C und ein oder mehrere optionale Rewards R an die Trainingsvorrichtung (bzw. Edge HW kommuniziert. Alternativ oder ergänzend kann, wenn der Controller (bzw. das Steuerungssystem) 200 selbst genügend Rechenressourcen zur Verfügung hat, das NN 516 direkt auf dem Controller (bzw. Steuerungssystem) 200 trainiert werden.

Ist das NN 516 hinreichend trainiert, wird in einem Ausführungsbeispiel die Policy auf den Controller (bzw. das Steuerungssystem) 200 übertragen. Dort wird dann damit der (z.B. im Wesentlichen konventionelle) Bewegungssteuerungsalgorithmus 304` zur Bestimmung der Ansteuerung C ersetzt oder adaptiert (wie beispielhaft an Bezugszeichen 622 in Fig. 6A mittels der Fusionseinheit bzw. "Aktion Fusion" illustriert).

Eine alternative oder ergänzende Möglichkeit besteht darin, die Signale S/O, A, C und R zu sammeln, optional anonymisiert an eine zentrale Instanz (und/oder Cloud) zu übertragen, und dort das Training der Policy durchzuführen, sofern die Rechenressourcen am Edge-System nicht ausreichend sind.

Eine weitere alternative oder ergänzende Möglichkeit besteht darin, das trainierte NN 516 (insbesondere umfassend die Policy) der Agenten an eine zentrale Instanz (und/oder Cloud) zu melden, und so die verschiedenen Policies der einzelnen medizintechnischen Komponenten (und/oder Geräte) statistisch miteinander abzugleichen. Somit kann eine noch generellere, für einen noch größeren Anwenderkreis gut funktionierende, Policy erlernt werden, die auf das Wissen aller Agenten im Feld rückgreift (sog. "Federated Learning"). Diese optimierte Policy kann z.B. zyklisch oder mit dem nächsten Software (SW)-Update an den Agenten (bzw. die medizintechnische Komponente bzw. das medizinische Gerät) zurückgespielt werden.

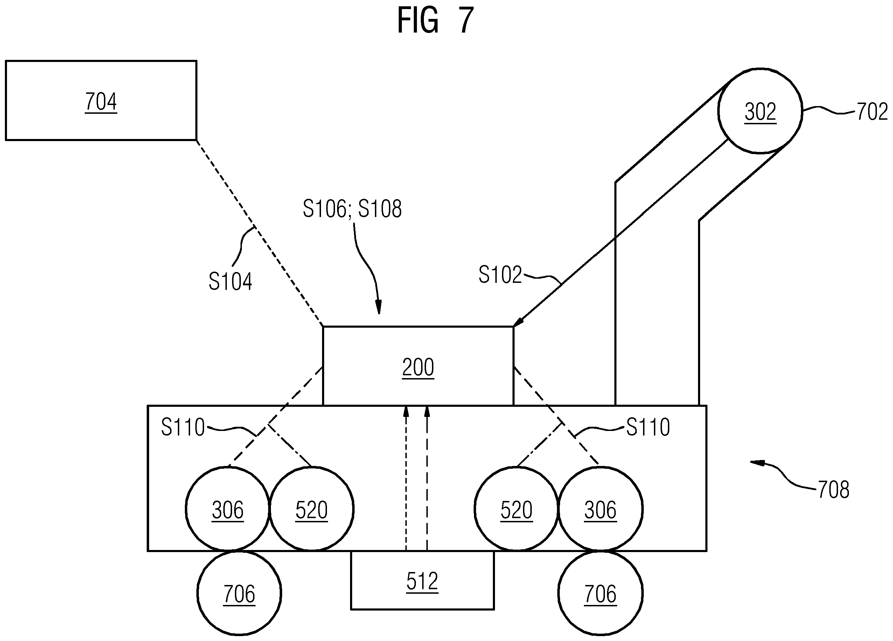

Fig. 7 zeigt schematisch eine medizintechnische Komponente 708 mit einem Handgriff 702. Der Handgriff 702 kann einen oder mehrere Kraftmesssensoren 302 umfassen, insbesondere zwei Kraftsensoren 302. Die erfassten S102 Kraftmessdaten werden vom Controller 200 empfangen, der die Antriebseinheiten der Räder 706 in Verkörperung von Motoren 306 mit Positionssensoren 520 ansteuert S110. Der Controller 200 kann mit Edge HW (und/oder einer Cloud) 704 kommunizieren, insbesondere über eine drahtlose Datenverbindung (z.B. Funk und/oder WLAN). Beispielsweise können über die drahtlose Datenverbindung mit der Edge HW (und/oder Cloud) 704 Umgebungsdaten von einem Umgebungssensorsystem empfangen werden, beispielsweise von am Einsatzort der medizintechnischen Komponente 708 installierten Kameras.

Die schematisch in Fig. 7 gezeigten Schritte S102; S104; S110 können jeweils Datenübertragungen (z.B. von Sensordaten und/oder Steuersignalen) umfassen.

Die Unterstützung des Bedieners kann deaktivierbar sein, um eine herkömmliche direkte Steuerung zu ermöglichen. In einem Ausführungsbeispiel kann die Aktivierung und/oder Deaktivierung intelligent ausgeführt sein, beispielsweise indem bei geringen Abweichungen kein Eingriff des ML-Algorithmus erfolgt. Die Abweichung kann sich beispielsweise auf eine optimierte und/oder sanfte Bewegung beziehen, und/oder auf einen optimierten Weg im Fall er Kenntnis des Zielorts.

Es ist nicht notwendigerweise zu erwarten, dass bei Verwendung von NNs 516 die "Policy" schon zur Entwicklungszeit hinreichend erlernt werden kann. Das bedeutet, dass erst wenn das Produkt (z.B. die medizintechnische Komponente 708 mit Controller 200) im Einsatz ist, der optimale Algorithmus erlernt wird. Um die Nutzung der erfindungsgemäßen Technik zu beschleunigen, kann ein Austausch der trainierten (auch; gelernten) NNs 516 zwischen den Produktinstanzen (z.B. baugleicher medizintechnischer Komponenten an verschiedenen Einsatzorten) möglich sein. Auch ein Austausch über Cloud-Instanzen ist denkbar.

Alternativ oder ergänzend kann der Einsatz der erfindungsgemäßen Technik beschleunigt werden, indem während der Entwicklung das NN 516 mit einem modell-basierten digitalen Zwilling der medizintechnischen Komponente (bzw. des Gerätes) 708 bereits trainiert wird. Dies hat den Vorteil, dass hiermit bereits tausende von Trainingsiterationen durchlaufen werden können, ehe die medizintechnische Komponente (bzw. das Gerät) zum Einsatz kommt.

Alternativ oder ergänzend umfasst ein Ausführungsbeispiel einen kontinuierlich lernenden Ansatz, da sich die Umgebung und HW-Eigenschaften über die Lebenszeit der medizintechnischen Komponente (bzw. des Produktes) verändern können. Auch sollte es möglich sein, die Lösung kundenspezifisch zu adaptieren, um kundenspezifische Lösungen anbieten zu können. So können beispielsweise spezifische Produktivitätsfeatures spezifisch freigegeben werden und/oder energieoptimierte Lösungen angeboten werden.

Eine alternative oder ergänzende Eigenschaft der erfindungsgemäßen Technik ist, dass die Handlungsspielräume des RL-Agenten im Steuerungssystem insgesamt beschränkbar sind. Dies kann essenziell sein, da ansonsten unvorhergesehene oder unkontrollierte Aktionen, z.B. Gerätebewegungen, entstehen könnten, was die Sicherheit des Bedienpersonals infrage stellen würde. Dies liegt in der Natur der RL-Algorithmen begründet, welche i.d.R. zumindest teilweise durch randomisierte Exploration lernen. Das bedeutet, dass nicht immer die gegenwärtig besten Aktionen der Policy verwendet werden, sondern zufällig gewählte Aktionen. Der Algorithmus probiert aus, ob dies zu noch besseren Rewards führt.

Beispielsweise kann der zulässige Aktionsspielraum des RL-Algorithmus auf 10% der aktuellen Motoransteuersignale begrenzt werden, so dass ein "Feintuning" durch den selbst lernenden Algorithmus möglich ist, das Großsignalverhalten jedoch weiterhin durch den (z.B. zumindest im Wesentlichen) konventionellen Bewegungssteuerungsalgorithmus (und/oder das konventionelle Steuerungssystem) dominiert wird. Hierfür kann, wie in Fig. 6A schematisch gezeigt, die Fusionseinheit (bzw. der Block Aktion Fusion) 622 vorgesehen sein.

Eine alternative oder ergänzende Anwendungsmöglichkeit der erfindungsgemäßen Technik besteht darin, die Rolle von "Agent" und "Environment" in der RL-Schleife (z.B. gedanklich) zu vertauschen. Das Nutzungsverhalten eines Benutzers der kraftgeführten Bewegung kann somit durch bestärkendes Lernen verbessert werden, insbesondere da RL biologisch inspiriert ist.

Man denke an einen Hund, der es durch "Leckerli" bekommen lernt, einen Stab zurückzubringen; oder an ein Kind, das durch "negativen" Reward (=Schmerz) erlernt, dass eine Herdplatte heiß und deshalb nicht anzufassen ist).

Im Fall der erfindungsgemäßen Technik kann die medizintechnische Komponente (bzw. das medizinische Gerät) durch eine Vorrichtung ergänzt werden, die dem Benutzer ein Reward-Signal übermittelt, und zwar dann, wenn die kraftunterstütze Benutzung der Komponente (bzw. des Geräts) gut gemacht wurde. Beispielsweise kann ein starkes Vibrationsfeedback bei zu starkem Kraftaufwand erfolgen (insbesondere als negatives Reward-Signal), oder eine grüne Beleuchtung, wenn die Krafteinwirkung bereits annähernd korrekt erfolgt. Auf diese Art und Weise kann der Lernprozess des Bedieners unterstützt und beschleunigt werden.

Alternativ oder ergänzend kann ein "inverse reinforcement learning" (IRL) angewendet werden, bei welchem der Reward erlernt wird. Hier kann dann folgendes Vorgehen angewendet werden. Zunächst werden Daten von einem oder mehreren erfahrenen Benutzern gesammelt. IRL wird eingesetzt, um eine Rewardfunktion zu lernen, die die erfahrene Benutzung codiert. Die Rewardfunktion wird auf dem RL-basierten System angewandt (auch: deployed). Die Rewardfunktion wird dann mit mindestens einer Policy trainiert.

Im Vergleich zu den konventionellen Ansätzen verwendet die erfindungsgemäße Technik einen lernenden Ansatz, um die Effizienz der kraftunterstützen Bewegungsführung zu erhöhen. Dies ermöglicht nach Abschluss der Lernphase eine allgemeingültige Unterstützung bei der Ansteuerung. Vorteilhafterweise kann die erfindungsgemäße Technik zu einer höheren Effizienz der medizinischen Workflows, einer höheren Akzeptanz der medizinischen Geräte und einer vereinfachten Bedienung auch durch ungeübtes Personal mit eingeschränkten Fähigkeiten ermöglichen.

Charakteristisch für die erfindungsgemäße Technik der kraftunterstützten Bewegungsführung einer medizintechnischen Komponente (bzw. eines medizinischen Geräts) sind beispielsweise die adaptive Anpassung an einen Benutzer sowie das Lernen der optimalen Bewegungsführung trotz unterschiedlicher Umgebung, Benutzer und/oder Alterung des Geräts.

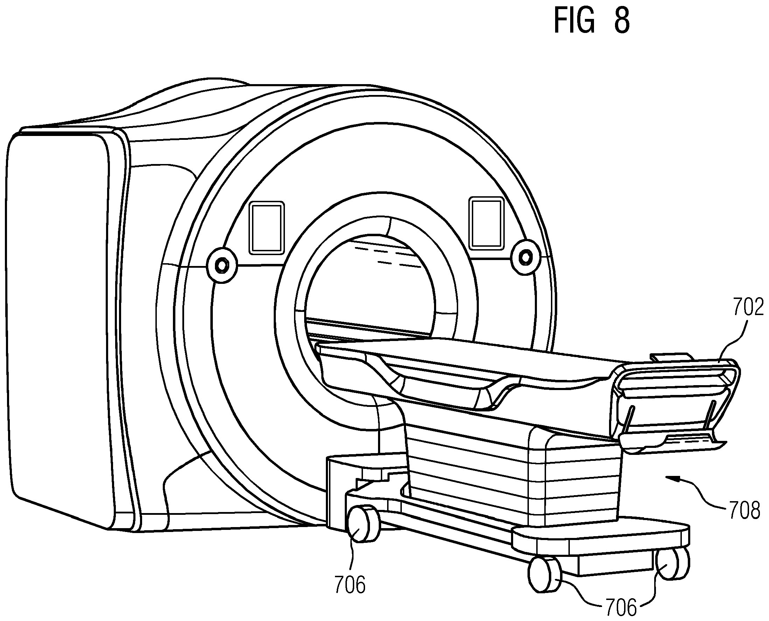

Fig. 8, 9, 10 und 11 zeigen Ausführungsbeispiele mobiler motorisch unterstützter medizintechnischer Komponenten 702 bzw. medizinischer Geräte, in denen die erfindungsgemäße Benutzersteuerung über einen Griff (auch: Kraftgriff) 702 erfolgt. Fig. 8 zeigt beispielhaft eine mobile Patientenliege (auch: mobiler MR-Tisch) 708, die zu einem MRT gehört. Die beispielhafte Patientenliege 708 hat vier Rollen 706 mit zumindest einer Antriebseinheit (nicht gezeigt) zur motorischen Unterstützung der Bewegung der Patientenliege 708.

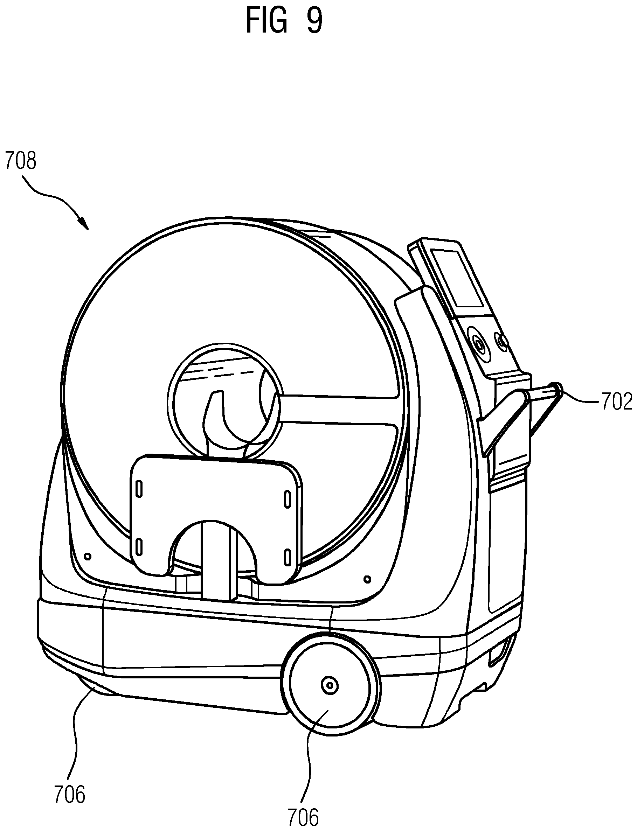

Fig. 9 zeigt beispielhaft einen mobilen CT 708 (z.B. einen Kopf-CT) mit Griff (auch: Kraftgriff) 702 und drei Rollen 706 mit zumindest einer Antriebseinheit (nicht gezeigt) zur motorischen Unterstützung des CT 708.

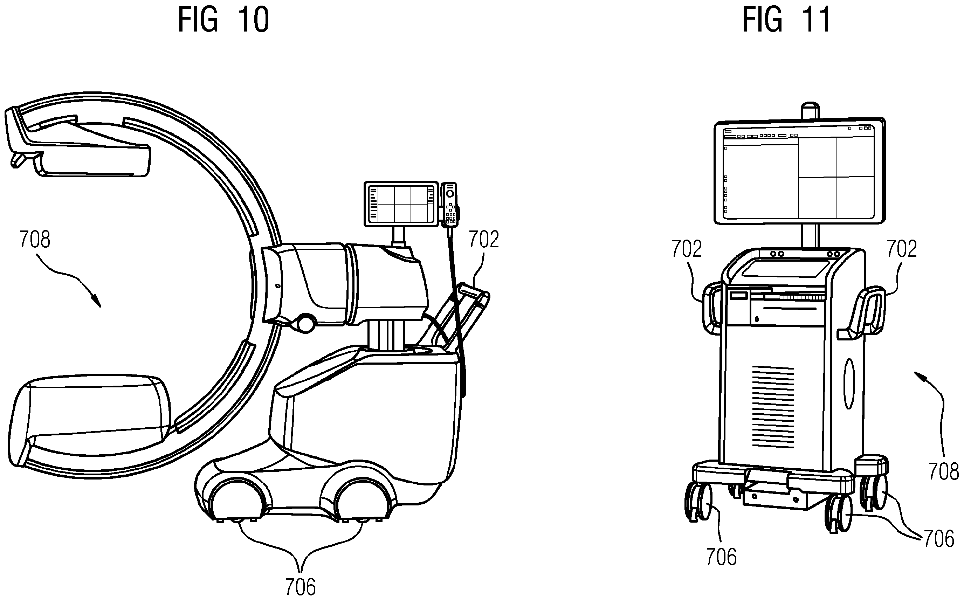

Figs. 10 und 11 zeigen einen mobilen C-Arm 708 bzw. ein mobiles Röntgengerät 708 mit einem bzw. zwei Griffen 702 und jeweils mehreren Rollen 706 mit mindestens einer Antriebseinheit (nicht gezeigt) zur motorischen Unterstützung der Bewegung.

Der Griff (auch: Kraftgriff) 702 kann in jedem Ausführungsbeispiel vorzugsweise mit zwei Kraftsensoren ausgestattet sein, womit die Richtung und Intensität des Bedienerwunsches erfasst werden können. Wenn zwei Sensoren nebeneinander angebracht sind, kann auch die Lenkung damit detektiert werden.

Eine Antriebsystem der motorischen Unterstützung kann in jedem Ausführungsbeispiel mittels Differentialgetriebe, holonomer Kinematik oder anderer herkömmlicher Kinematik realisiert werden.

Eine Lokalisierungssensorik (und/oder das Umgebungssensorsystem) kann einen oder mehrere LIDAR Sensoren, 3D-Kameras, Radarsensoren und/oder andere geeignete Sensoren umfassen.

Ein Ziel der Lokalisierungssensorik ist es, die absolute Pose der mobilen medizintechnischen Komponente (bzw. des medizinischen Geräts) zu schätzen auf Basis einer Umgebungskarte (auch; 2D Karte) des Einsatzortes (z.B. eines Krankenhauses). Alternativ oder ergänzend kann eine Simultane Positionsbestimmung und Kartierung (fachsprachlich: Simultaneous Localization and Mapping, SLAM) ausgeführt bzw. eine Schätzung und Mapping zusammen gemacht werden.

Die Lokalisierungssensorik (und/oder das Umgebungssensorsystem) kann gemäß der erfindungsgemäßen Technik für mehrere Ziele eingesetzt werden.

Die Lokalisierungssensorik (und/oder das Umgebungssensorsystem) kann zur Ermittlung der absoluten Pose der mobilen medizintechnischen Komponente (bzw. des medizinischen Geräts) eingesetzt werden. Hierdurch kann die Intention des Benutzers mit der Position in der Karte in Verbindung gesetzt werden. Beispielsweise kann die medizintechnische Komponente (bzw. das Gerät) auf einem Flur nahe einer Tür auf der rechten Seite sein und der Bediener eine Krafteinwirkung nach rechts applizieren. Somit kann angenommen werden, dass der Bediener durch die Tür fahren will. Dies kann das NN während der Trainingsphase erlernen und später (und/ oder in der Inferenzphase) den optimalen Weg durch die Tür ausgeben.

Alternativ oder ergänzend kann die Karte vor dem Training (z.B. von medizinischem und/oder technischem Personal) annotiert werden, um beispielsweise Behandlungsräume oder kritische Stellen zu markieren. Die Annotationen der Karte können während der Trainingsphase mit einbezogen werden.

Weiterin alternativ oder ergänzend kann die Lokalisierungssensorik (und/oder das Umgebungssensorsystem) zur Ermittlung von unbekannten Objekten während des Betriebs verwendet werden, insbesondere um Kollisionen mit mobilen Hindernissen zu vermeiden. Beispielsweise kann eine Bewegung unabhängig von den erfassten Kraftmessdaten verlangsamt oder gestoppt werden, wenn ein mobiles Hindernis in Bewegungsrichtung der medizintechnischen Komponente erkannt wird,
Unabhängig vom grammatikalischen Geschlecht eines bestimmten Begriffes sind Personen (z.B. Patienten und/oder medizinisches Personal) mit männlicher, weiblicher oder anderer Geschlechteridentität mit umfasst.

Soweit nicht bereits explizit beschrieben, können einzelne Ausführungsformen oder deren einzelne Aspekte und Merkmale, die in Bezug auf die Zeichnungen beschrieben sind, miteinander kombiniert oder ausgetauscht werden, ohne den Umfang der beschriebenen Erfindung einzuschränken oder zu erweitern, wenn eine solche Kombination oder ein solcher Austausch sinnvoll und im Sinne der vorliegenden Erfindung ist. Vorteile, die in Bezug auf eine bestimmte Ausführungsform der vorliegenden Erfindung oder in Bezug auf eine bestimmte Figur beschrieben sind, sind, wo immer dies zutrifft, auch Vorteile anderer Ausführungsformen der vorliegenden Erfindung.

## Patentansprüche

1. Computer-implementiertes Verfahren (100) zum Ansteuern einer Antriebseinheit (306) zur motorischen Unterstützung einer Bewegung einer medizintechnischen Komponente (708), umfassend die Schritte:
- Erfassen (S102) von Kraftmessdaten, die eine Kraft repräsentieren, die zur Bewegungssteuerung einer mobilen motorisch unterstützten medizintechnischen Komponente (708) an einem Bedienelement, insbesondere einem Handgriff (702), an der medizintechnischen Komponente (708) aufgebracht wird;
- Verarbeiten (S106), mittels eines neuronalen Netzwerks, der erfassten (S102) Kraftmessdaten zum Bestimmen (S108) von Steuerdaten zur Ansteuerung der Antriebseinheit (306) zur motorischen Unterstützung einer Bewegung der medizintechnischen Komponente (708); und
- Ansteuern (S110) der Antriebseinheit (306) mittels den bestimmten (S108) Steuerdaten.

2. Verfahren (100) gemäß Anspruch 1, wobei die Kraftmessdaten von einem Kraftmesssensorsystem erfasst werden, das an dem Bedienelement angeordnet ist und zumindest einen Sensor umfasst, der ausgewählt ist aus der folgenden Gruppe, bestehend aus:
- einem Kraftsensor;
- einem Berührungssensor; und
- einem Richtungssensor.

3. Verfahren (100) gemäß einem der vorhergehenden Ansprüche, ferner umfassend den Schritt:
- Empfangen (S104) von Umgebungsdaten hinsichtlich einer Umgebung der medizintechnischen Komponente (708) mittels eines Umgebungssensorsystems;
wobei ein Verarbeiten (S106) der Umgebungsdaten und das Bestimmen (S108) der Steuerdaten ferner auf den empfangenen (S104) Umgebungsdaten basiert.

4. Verfahren (100) gemäß einem der vorhergehenden Ansprüche, wobei das Bestimmen (S108) der Steuerdaten ferner auf einer gespeicherten Umgebungskarte basiert.

5. Verfahren (100) gemäß einem der vorhergehenden Ansprüche, wobei der Schritt des Bestimmens (S108) von Steuerdaten zumindest teilweise von dem neuronalen Netzwerk ausgeführt wird.

6. Verfahren (100) gemäß einem der vorhergehenden Ansprüche, wobei das neuronale Netzwerk mittels maschinellen Lernens, ML, insbesondere bestärkendem Lernen, RL, trainiert ist.

7. Verfahren (100) gemäß einem der vorhergehenden Ansprüche, wobei zum Trainieren des neuronalen Netzwerkes ein Belohnungssystem verwendet wird, das mindestens eine der folgenden Eigenschaften einer Bewegung belohnt:
- eine sanfte Bewegung;
- ein Einhalten eines vorbestimmten Wegs;
- ein Einhalten einer Zeitvorgabe;
- ein Einhalten einer vorgegebenen Lautstärke; und/oder
- eine Kollisionsfreiheit.

8. Verfahren (100) gemäß einem der vorhergehenden Ansprüche, wobei der Schritt des Bestimmens (S108) von Steuerdaten zumindest teilweise von einem Bewegungssteuerungsalgorithmus ausgeführt wird, wobei der Bewegungssteuerungsalgorithmus eine vorbestimmte Parametrisierung der Bewegung in Abhängigkeit der empfangenen (S102) Kraftmessdaten umfasst.

9. Verfahren (100) gemäß einem der vorhergehenden Ansprüche, wobei das neuronale Netzwerk anhand von simulierten und/oder historischen Bewegungsdaten der medizintechnischen Komponente (708) trainiert wird.

10. Verfahren (100) gemäß einem der vorhergehenden Ansprüche, wobei die Ansteuerung der Antriebseinheit (306) zur motorischen Unterstützung deaktivierbar ist.

11. Verfahren (100) gemäß einem der vorhergehenden Ansprüche, ferner umfassend den Schritt:
- Ausgeben eines Signals hinsichtlich einer Qualität der Bewegungssteuerung durch die erfassten (S102) Kraftmessdaten, wobei die Qualität im Schritt des Verarbeitens (S106) der Kraftmessdaten durch das neuronale Netzwerk ermittelt wird.

12. Controller (200) zum Ansteuern einer Antriebseinheit (306) zur motorischen Unterstützung einer Bewegung einer medizintechnischen Komponente (708), umfassend:
- eine Kraftdatenerfassungseinheit (202), die zum Erfassen von Kraftmessdaten ausgebildet ist, die eine Kraft repräsentieren, die zur Bewegungssteuerung einer mobilen motorisch unterstützten medizintechnischen Komponente (708) an einem Bedienelement, insbesondere einem Handgriff (702), an der medizintechnischen Komponente (708) aufgebracht wird;
- eine Verarbeitungseinheit (206), die dazu ausgebildet ist, mittels eines neuronalen Netzwerks die erfassten Kraftmessdaten zu verarbeiten;
- eine Bestimmungseinheit (208), die zum Bestimmen von Steuerdaten zur Ansteuerung einer Antriebseinheit (306) zur motorischen Unterstützung einer Bewegung der medizintechnischen Komponente (708) ausgebildet ist; und
- eine Ansteuerungsschnittstelle (210), die dazu ausgebildet ist, die Antriebseinheit (306) mittels den bestimmten Steuerdaten anzusteuern.

13. System zum Ansteuern einer Antriebseinheit (306) zur motorischen Unterstützung einer Bewegung einer medizintechnischen Komponente (708), umfassend:
- einen Controller (200) gemäß dem direkt vorhergehenden Anspruch; und
- mindestens eine medizintechnische Komponente (708) mit mindestens einer Antriebseinheit (306) zur motorischen Unterstützung der Bewegung.

14. Computerprogrammprodukt mit Programmelementen, die einen Controller (200) veranlassen, die Schritte des Verfahrens zum Ansteuern einer Antriebseinheit (306) zur motorischen Unterstützung einer Bewegung einer medizintechnischen Komponente (708) nach einem der vorhergehenden Verfahrensansprüche auszuführen, wenn die Programmelemente in einen Speicher des Controllers (200) geladen werden.

15. Computerlesbares Medium, auf dem Programmelemente gespeichert sind, die von einem Controller (200) gelesen und ausgeführt werden können, um Schritte des Verfahrens zum Ansteuern einer Antriebseinheit (306) zur motorischen Unterstützung einer Bewegung einer medizintechnischen Komponente (708) gemäß einem der vorangehenden Verfahrensansprüche durchzuführen, wenn die Programmelemente von dem Controller (200) ausgeführt werden.
